# EUROPÄISCHE PATENTANMELDUNG

(11) **EP 1 717 291 A2**
(43) Veröffentlichungstag der Anmeldung: **02.11.2006**
(21) Anmeldenummer: 06014637.0
(22) Anmeldetag: 13.04.2004
(51) Int. Cl.: C09K 11/06, H05B 33/14, C07C 49/223, C07C 49/417, C07C 49/67, C07C 49/675, C07C 49/697, C07C 49/784, C07C 49/792, C07C 49/796, C07C 49/798, C07C 49/813, C07C 49/84, C07C 50/02, C07C 251/24, C07C 325/02, C07C 251/20

(54) **Mischungen von organischen, zur Emission befähigten Halbleitern und Maxtrixmaterialien, deren Verwendung und diese Mischungen enthaltende Elektronikbauteile**

(30) Priorität: 15.04.2003 DE 10317556; 25.11.2003 DE 10355358
(62) Teilanmeldung aus: 04726968.3
(71) Anmelder: Merck Patent GmbH, 64293 Darmstadt (DE)
(72) Erfinder: Gerhard, Anja, Dr., 64291 Darmstadt (DE); Vestweber, Horst, Dr., 34630 Gilersberg-Winterscheid (DE); Stössel, Philipp, Dr., 60487 Frankfurt am Main (DE); Heun, Susanne, Dr., 65812 Bad Soden (DE); Spreitzer, Hubert, Dr., 68519 Viernheim (DE)

(57) **Zusammenfassung**

Mischungen von organischen zur Emission befähigten Halbleitern und Matrixmaterialien, deren Verwendung und Elektronikbauteile enthaltend diese

Die vorliegende Erfindung beschreibt neuartige Materialmischungen aus mindestens zwei Substanzen, wobei die eine als Matrixmaterial dient und die andere ein zur Emission befähigtes Emissionsmaterial ist, welches mindestens ein Element der Ordungszahl größer 20 enthält, und deren Verwendung in organischen elektronischen Bauteilen wie Elektrolumineszenzelementen und Displays.

## Beschreibung

Die vorliegende Erfindung beschreibt die Verwendung neuer Materialien und Materialmischungen in organischen elektronischen Bauteilen wie Elektrolumineszenzelementen und deren Verwendung in darauf basierenden Displays.

in einer Reihe verschiedenartiger Anwendungen, die im weitesten Sinne der Elektronikindustrie zugerechnet werden können, ist der Einsatz organischer Halbleiter als Wirkkomponenten (= Funktionsmaterialien) seit geraumer Zeit Realität bzw. wird in naher Zukunft erwartet. So finden schon seit etlichen Jahren lichtsensitive organische Materialien (z. B. Phthalocyanine) sowie organische Ladungstransportmaterialien (i. d. R. Lochtransporter auf Triarylaminbasis) Verwendung in Kopiergeräten. Der Einsatz spezieller halbleitender organischer Verbindungen, die zur Emission von Licht im sichtbaren Spektralbereich befähigt sind, steht gerade am Anfang der Markteinführung, zum Beispiel in organischen Elektrolumineszenzvorrichtungen. Deren Einzelbauteile, die organischen lichtemittierenden Dioden (OLEDs), besitzen ein sehr breites Anwendungsspektrum als:
1. weiße oder farbige Hinterleuchtungen für monochrome oder mehrfarbige Anzeigeelemente (wie z. B. im Taschenrechner, für Mobiltelefone und andere tragbare Anwendungen),
2. großflächige Anzeigen (wie z. B. Verkehrsschilder, Plakate und andere Anwendungen),
3. Beleuchtungselemente in allen Farben und Formen,
4. monochrome oder vollfarbige Passiv-Matrix-Displays für tragbare Anwendungen (wie z. B. Mobiltelefone, PDAs, Camcorder und andere Anwendungen),
5. vollfarbige, großflächige, hochauflösende Aktiv-Matrix-Displays für verschiedenste Anwendungen (wie z. B. Mobiltelefone, PDAs, Laptops, Fernseher und andere Anwendungen).

Bei diesen Anwendungen ist die Entwicklung teilweise bereits sehr weit fortgeschritten; dennoch besteht immer noch großer technischer Verbesserungsbedarf.

Für einfachere OLEDs enthaltende Vorrichtungen ist die Markteinführung bereits erfolgt, wie die Autoradios der Firma Pioneer oder eine Digitalkamera der Firma Kodak mit "Organischem Display" belegen. Allerdings gibt es immer noch erhebliche Probleme, die einer dringenden Verbesserung bedürfen:
1. So ist v. a. die operative Lebensdauer von OLEDs immer noch gering, so dass bis dato nur einfache Anwendungen kommerziell realisiert werden können.
2. Die Effizienzen von OLEDs sind zwar akzeptabel, aber auch hier sind ― gerade für tragbare Anwendungen ("portable applications") ― immer noch Verbesserungen erwünscht.
3. Die Farbkoordinaten von OLEDs, speziell im Roten, sind nicht gut genug. Besonders die Kombination von guten Farbkoordinaten mit hoher Effizienz muss noch verbessert werden.
4. Die Alterungsprozesse gehen i. d. R. mit einem Anstieg der Spannung einher. Dieser Effekt macht spannungsgetriebene organische Elektrolumineszenzvorrichtungen, z. B. Displays oder Anzeige-Elemente, schwierig bzw. unmöglich. Eine stromgetriebene Ansteuerung ist aber gerade in diesem Fall aufwendiger und teurer.
5. Die benötigte Betriebsspannung ist gerade bei effizienten phosphoreszierenden OLEDs recht hoch und muss daher verringert werden, um die Leistungseffizienz zu verbessern. Das ist gerade für tragbare Anwendungen von großer Bedeutung.
6. Der benötigte Betriebsstrom ist ebenfalls in den letzten Jahren verringert worden, muss aber noch weiter verringert werden, um die Leistungseffizienz zu verbessern. Das ist gerade für tragbare Anwendungen besonders wichtig.
7. Durch die Vielfalt an Schichten ist der Aufbau der OLEDs komplex und technologisch sehr aufwendig. Daher wäre es wünschenswert, OLEDs mit einem einfacheren Schichtaufbau, der weniger Schichten benötigt, mit weiterhin guten oder sogar verbesserten Eigenschaften realisieren zu können.

Die oben unter 1. bis 7. genannten Gründe machen Verbesserungen bei der Herstellung von OLEDs notwendig.

Eine Entwicklung hierzu, die sich in den letzten Jahren abzeichnet, ist der Einsatz metallorganischer Komplexen, die Phosphoreszenz statt Fluoreszenz zeigen [M. A. Baldo, S. Lamansky, P. E. Burrows, M. E. Thompson, S. R. Forrest, Appl. Phys. Lett. 1999, 75, 4-6]. Aus quantenmechanischen Gründen ist unter Verwendung metallorganischer Verbindungen eine bis zu vierfache Quanten-, Energie- und Leistungseffizienz möglich. Ob sich diese neue Entwicklung durchsetzen wird, hängt zum einen stark davon ab, ob entsprechende Device-Kompositionen gefunden werden können, die diese Vorteile (Triplett-Emission = Phosphoreszenz gegenüber Singulett-Emission = Fluoreszenz) auch in OLEDs umsetzen können. Als wesentliche Bedingungen für die praktische Anwendung sind hier insbesondere eine hohe operative Lebensdauer, eine hohe Stabilität gegenüber Temperaturbelastung und eine niedrige Einsatz- und Betriebsspannung, um mobile Applikationen zu ermöglichen, zu nennen.

Der allgemeine Aufbau von organischen Elektrolumineszenzvorrichtungen ist beispielsweise in US 4,539,507 und US 5,151,629, sowie EP 01202358 beschrieben.

Üblicherweise besteht eine organische Elektrolumineszenzvorrichtung aus mehreren Schichten, die mittels Vakuummethoden oder unterschiedlicher Druckmethoden aufeinander aufgebracht werden. Diese Schichten sind im einzelnen:
1. Eine Trägerplatte = Substrat (üblicherweise Glas oder Kunststofffolien).
2. Eine transparente Anode (üblicherweise Indium-Zinn-Oxid, ITO).
3. Eine Lochinjektionsschicht (Hole Injection Layer = HIL): z. B. auf Basis von Kupter-phthalocyanin (CuPc) oder leitfähigen Polymeren, wie Polyanilin (PANI) oder Polythiophen-Derivaten (wie PEDOT).
4. Eine oder mehrere Lochtransportschichten (Hole Transport Layer = HTL): üblicherweise auf Basis von Triarylamin-Derivaten, z. B. 4,4',4"-Tris(N-1-naphthyl-N-phenyl-amino)-triphenylamin (NaphDATA) als erste Schicht und N,N'-Di(naphth-1-yl)-N,N'-diphenyl-benzidin (NPB) als zweite Lochtransportschicht.
5. Eine oder mehrere Emissionsschichten (Emission Layer = EML): diese Schicht (bzw. Schichten) kann teilweise mit den Schichten 4 bis 8 zusammenfallen, besteht aber üblicherweise aus mit Fluoreszenzfarbstoffen, z. B. N,N'-Diphenyl-chinacridon (QA), oder Phosphoreszenzfarbstoffen, z. B. Tris(2-phenylpyridyl)-iridium (Ir(PPy)₃) oder Tris(2-benzothiophenyl-pyridyl)-iridium (Ir(BTP)₃), dotierten Matrixmaterialien, wie 4,4'-Bis(carbazol-9-yl)-biphenyl (CBP). Die Emissions-Schicht kann aber auch aus Polymeren, Mischungen von Polymeren, Mischungen von Polymeren und niedermolekularen Verbindungen oder Mischungen verschiedener niedermolekularer Verbindungen bestehen.
6. Eine Lochblockierschicht (Hole-Blocking-Layer = HBL): diese Schicht kann teilweise mit den Schichten 7 und 8 zusammenfallen. Sie besteht üblicherweise aus BCP (2,9-Dimethyl-4,7-diphenyl-1,10-phenanthrolin = Bathocuproin) oder Bis-(2-methyl-8-chinolinolato)-(4-phenyl-phenolato)-aluminium(III) (BAlq).
7. Eine Elektronentransportschicht (Electron Transport Layer = ETL): meist auf Basis von Aluminium-tris-8-hydroxychinolinat (AIQ₃).
8. Eine Elektroneninjektionsschicht (Electron Injection Layer = EIL): diese Schicht kann teilweise mit Schicht 4, 5, 6 und 7 zusammenfallen, bzw. es wird ein kleiner Teil der Kathode speziell behandelt bzw. speziell abgeschieden.
9. Eine weitere Elektroneninjektionsschicht (Electron Injection Layer = EIL): eine dünne Schicht bestehend aus einem Material mit einer hohen Dielektrizitätskonstanten, wie z. B. LiF, Li₂O, BaF₂, MgO, NaF.
10. Eine Kathode: hier werden in der Regel Metalle, Metallkombinationen oder Metallegierungen mit niedriger Austrittsarbeit verwendet, so z. B. Ca, Ba, Cs, Mg, Al, In, Mg/Ag.

Diese ganze Vorrichtung wird entsprechend (je nach Anwendung) strukturiert, kontaktiert und schließlich auch hermetisch versiegelt, da sich die Lebensdauer derartiger Vorrichtungen bei Anwesenheit von Wasser und/oder Luft drastisch verkürzt. Das Gleiche gilt auch für sogenannte invertierte Strukturen, bei denen das Licht aus der Kathode ausgekoppelt wird. Bei diesen invertierten OLEDs besteht die Anode z. B. aus Al/Ni/NiOx oder Al/Pt/PtOx oder anderen Metall/Metalloxid-Kombinationen, die ein HOMO größer 5 eV besitzen. Die Kathode besteht dabei aus den gleichen Materialien, die in Punkt 9 und 10 beschrieben sind, mit dem Unterschied, dass das Metall, wie z. B. Ca, Ba, Mg, Al, In usw., sehr dünn und damit transparent ist. Die Schichtdicke liegt unter 50 nm, besser unter 30 nm, noch besser unter 10 nm. Auf diese transparente Kathode kann noch ein weiteres transparentes Material aufgebracht werden, z. B. ITO (Indium-Zinn-Oxid), IZO (Indium-Zink-Oxid), usw.

Im oben genannten Aufbau kommt dem Matrixmaterial der Emissionsschicht (EML) eine besondere Rolle zu. Das Matrixmaterial muss den Ladungstransport von Löchern und/oder Elektronen ermöglichen oder verbessern und/oder die Ladungsträgerrekombination ermöglichen oder verbessern und gegebenenfalls die bei der Rekombination entstehende Energie auf den Emitter übertragen. Diese Aufgabe wird bei den Elektrolumineszenzvorrichtungen auf Basis phosphoreszierender Emitter bislang überwiegend von Matrixmaterialien, die Carbazol-Einheiten enthalten, übernommen.

Matrixmaterialien, die Carbazoleinheiten, wie z. B. das häufig verwendete 4,4'-Bis-(N-carbazolyl)-biphenyl (CBP), enthalten, haben in der Praxis jedoch einige Nachteile. Diese sind unter anderem in der oftmals kurzen bis sehr kurzen Lebensdauer der mit ihnen hergestellten Devices und den häufig hohen Betriebsspannungen, die zu geringen Leistungseffizienzen führen, zu sehen. Des weiteren hat sich gezeigt, dass aus energetischen Gründen CBP für blau emittierende Elektrolumineszenzvorrichtungen ungeeignet ist, was in einer schlechten Effizienz resultiert. Außerdem ist der Aufbau der Devices sehr komplex, wenn CBP als Matrixmaterial verwendet wird, da zusätzlich eine Lochblockierschicht und eine Elektronentransportschicht verwendet werden müssen. Werden diese zusätzlichen Schichten nicht verwendet, wie z. B. von Adachi et al. (Organic Electronics 2001, 2, 37) beschrieben, so beobachtet man zwar gute Effizienzen, aber nur bei extrem geringen Helligkeiten, während die Effizienz bei höherer Helligkeit, wie sie für die Anwendung nötig ist, um mehr als eine Größenordnung geringer ist. So werden für hohe Helligkeiten hohe Spannungen benötigt, so dass hier die Leistungseffizienz sehr niedrig ist, was insbesondere für Passiv-Matrix-Anwendungen ungeeignet ist.

Es wurde nun überraschend gefunden, dass die Verwendung bestimmter Matrixmaterialien in Kombination mit bestimmten Emittern zu deutlichen Verbesserungen gegenüber dem Stand der Technik, insbesondere in Bezug auf die Effizienz und in Kombination mit einer stark erhöhten Lebensdauer, führen. Zudem ist mit diesen Matrixmaterialien ein deutlich vereinfachter Schichtaufbau der OLED möglich, da weder eine separate Lochblockierschicht, noch eine separate Elektronentransport- und/oder Elektroneninjektionsschicht verwendet werden muss. Dies ist ein enormer technologischer Vorteil.

Die Verwendung der nachfolgend beschriebenen Matrixmaterialien in OLEDs, die phosphoreszierende Emitter enthalten, ist ebenso neu wie die zugrundeliegende Mischung.

Die Verwendung analoger Materialien in einfachen Devices, als Emissionsmaterialien selbst oder als Materialien in der Emissionsschicht in Kombination mit fluoreszierenden Emittern ist vereinzelt schon in der Literatur beschrieben worden (z. B. JP 06192654). Ebenso gibt es eine Beschreibung (WO 04/013080) von Aroyl-Derivaten des Spirobifluorens, die auch in OLEDs verwendet werden können, allerdings ohne Bezug auf Triplett-Emission, Elektrophosphoreszenz oder Matrixmaterialien dafür; diese ist somit als zufällige Offenbarung zu bewerten. Die nachfolgend beschriebene Erfindung wird von den oben genannten Beschreibungen nicht neuheitsschädlich berührt, da die Verwendung der nachfolgend beschriebenen Matrixmaterialien in OLEDs in Kombination mit phosphoreszierenden Emittern neu ist.

Gegenstand der Erfindung sind deshalb Mischungen enthaltend
- mindestens ein Matrixmaterial A, welches eine Struktureinheit der Form C=Q enthält, bei dem Q mindestens ein nicht-bindendes Elektronenpaar aufweist und für das Element O, S, Se oder N steht, und welches gegebenenfalls auch glasartige Schichten bilden kann, und
- mindestens ein zur Emission befähigten Emissionsmaterial B, welches eine Verbindung ist, die bei geeigneter Anregung Licht emittiert und mindestens ein Element der Ordungszahl größer 20 enthält.

Bevorzugt handelt es sich bei den erfindungsgemäßen Mischungen um solche, die mindestens ein Matrixmaterial A enthalten, bei dem die Glastemperatur T_{g} der Reinsubstanz A größer 70 °C ist, bevorzugt größer 100 °C, besonders bevorzugt größer 130 °C.

Die oben beschriebenen Mischungen enthalten bevorzugt als Matrixmaterial A mindestens eine Verbindung gemäß Formel (1), Formel (2) und/oder Formel (3) 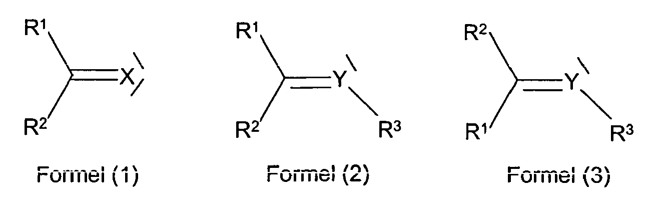 wobei die Symbole und Indizes folgende Bedeutung haben:
- X: ist bei jedem Auftreten gleich oder verschieden O, S oder Se;
- Y: ist bei jedem Auftreten N;
- R¹, R², R³: ist gleich oder verschieden bei jedem Auftreten H, CN, eine geradkettige, verzweigte oder cyclische Alkyl-, Alkoxy-oder Alkylaminogruppe mit 1 bis 40 C-Atomen, wobei ein oder mehrere nicht benachbarte CH₂-Gruppen durch -R⁴C=CR⁴-, -C≡C-, C=O, C=S, C=Se, C=NR⁴, -O-, -S-, -NR⁵- oder -CONR⁶- ersetzt sein können und wobei ein oder mehrere H-Atome durch F, Cl, Br, I ersetzt sein können,oder
ein aromatisches oder heteroaromatisches System mit 1 bis 40 C-Atomen, wobei ein oder mehrere H-Atome durch F, Cl, Br, I ersetzt sein können und das durch einen oder mehrere, nicht aromatische Reste R¹ substituiert sein kann, wobei mehrere Substituenten R¹ und/oder R¹, R² sowohl am selben Ring als auch an den beiden unterschiedlichen Ringen zusammen wiederum ein weiteres mono- oder polycyclisches, aliphatisches oder aromatisches Ringsystem aufspannen können; mit der Maßgabe, dass R¹=R²=R³ ungleich Wasserstoff ist;
- R⁴, R⁵, R⁶: sind gleich oder verschieden bei jedem Auftreten H oder ein aliphatischer oder aromatischer Kohlenwasserstoffrest mit 1 bis 20 C-Atomen.

Unter einem aromatischen bzw. heteroaromatischen System im Sinne dieser Erfindung soll ein System verstanden werden, das nicht notwendigerweise nur aromatische bzw. heteroaromatische Gruppen enthält, sondern in dem auch mehrere aromatische bzw. heteroaromatische Gruppen durch eine kurze nicht-aromatische Einheit (< 10 % der Atome, bevorzugt < 5 % der Atome), wie beispielsweise sp³-hybridisierter C, O, N, etc., unterbrochen sein können. So sollen also beispielsweise auch Systeme wie 9,9'-Spirobifluoren, 9,9-Diarylfluoren, Triarylamin, Diphenylether, etc. als aromatische Systeme verstanden werden.

Auch wenn dies aus der Definition oben hervorgeht, sei hier nochmals explizit darauf verwiesen, dass der Rest R¹ bzw. R² auch eine substituierte oder unsubstituierte Vinylgruppe bzw. ein entsprechendes Derivat sein kann, d. h. dass die Verbindung gemäß Formel (1) auch eine α,β-ungesättigte Carbonyl-Verbindung sein kann, bzw. die Verbindung gemäß Formel (2) oder (3) auch ein α,β-ungesättigtes Imin sein können.

Als besonders geeignete Verbindungen gemäß Formel (1) bis (3) haben sich Verbindungen erwiesen, die nicht planar aufgebaut sind. An der Struktureinheit der Form C=Q können entsprechende Substituenten für eine Abweichung der Gesamtstruktur von der Planarität sorgen. Dies ist insbesondere dann der Fall, wenn mindestens einer der Substituenten R¹, R² und/oder R³ wenigstens ein sp³-hybridisiertes Kohlenstoff-, Silicium-, Germanium- und/oder Stickstoffatom enthält, welches dadurch näherungsweise tetraedrische oder im Fall von Stickstoff pyramidale Bindungsgeometrie aufweist.

Um eine deutliche Abweichung von der Planarität zu erreichen, ist es bevorzugt, wenn wenigstens eines der sp³-hybridisierten Atome ein sekundäres, tertiäres oder quartäres Atom ist, besonders bevorzugt ein tertiäres oder quartäres Atom, im Fall von Kohlenstoff, Silicium oder Germanium ganz besonders bevorzugt ein quartäres Atom ist.

Unter einem sekundären, tertiären oder quartären Atom wird ein Atom mit zwei, drei bzw. vier Substituenten ungleich Wasserstoff verstanden.

Bevorzugt sind weiterhin Verbindungen, die in mindestens einem der Reste R¹ bis R³ ein 9,9'-Spirobifluorenderivat, bevorzugt verknüpft über die 2- und/oder 2,7- und/oder 2,2'-und/oder 2,2',7- und/oder 2,2',7,7'-Position, ein 9,9-disubstituiertes Fluorenderivat, bevorzugt verknüpft über die 2- und/oder 2,7-Position, ein 6,6- und/oder 12,12-di- oder tetrasubstituiertes Indenofluorenderivat, ein Triptycenderivat, bevorzugt verknüpft über die 9- und/oder 10-Position, ein Dihydrophenanthren-Derivat, bevorzugt verknüpft über die 2- und/oder 2,7- und/oder 3-und/oder 3,6-Position, oder ein Hexaarylbenzolderivat, bevorzugt verknüpft über die p-Position am bzw. an den Aromaten, enthalten.

Besonders bevorzugt sind Verbindungen, die in mindestens einem der Reste R¹ bis R³ ein 9,9'-Spirobifluorenderivat enthalten.

Nochmals weiterhin bevorzugt sind Verbindungen, die in mindestens einem der Reste R¹ bis R³ ein substituiertes oder unsubstituiertes 2-Biphenyl bzw. einen substituierten oder unsubstituierten 2-Biphenylether enthalten.

Weiterhin bevorzugt sind Verbindungen, die dendritisch aufgebaut sind. Außerdem bevorzugt sind 1,3,5-trisubstituierte Benzolketone und entsprechende Oligoketone, die beispielsweise gemäß N. Nakamura et al., J. Amer. Chem. Soc. 1992, 114, 1484, oder gemäß K. Matsuda et al., J. Amer. Chem. Soc. 1995, 117, 5550, erhältlich sind.

Um Mißverständnissen vorzugbeugen, sei an dieser Stelle betont, dass mit Matrixmaterialien A mit der Struktureinheit C=Q natürlich keine aromatischen Systeme, die partielle C=N-Doppelbindungen im Ring enthalten, wie z. B. Pyrimidine, Pyrazine, etc., gemeint sind.

Ebenso bevorzugt sind Mischungen, die als Matrixmaterial A mindestens eine Verbindung gemäß Formel (4) bis (9) enthalten, 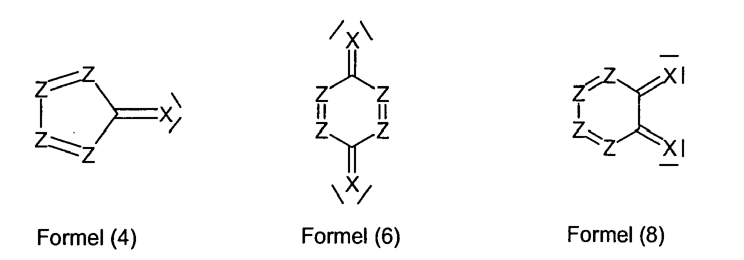 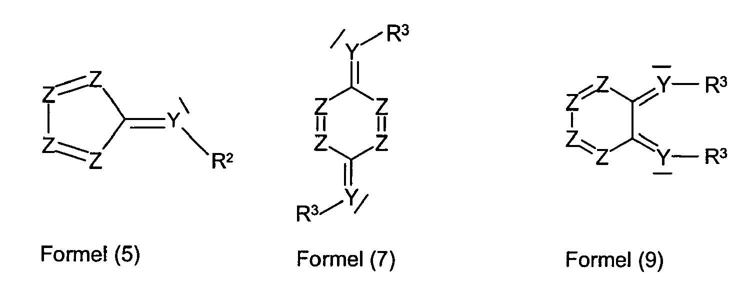 wobei die Symbole X, Y, R¹, R², R³, R⁴, R⁵ und R⁶ die unter den Formeln (1) bis (3) genannten Bedeutungen haben, und
- Z: gleich oder verschieden bei jedem Auftreten CR¹ oder N ist.

Besonders bevorzugt sind organische Mischungen, die mindestens eines der oben durch Formel (1) bis (9) beschriebenen Matrixmaterialien A enthalten, bei welchem gilt:
- X: ist bei jedem Auftreten O oder S;
- Y: ist bei jedem Auftreten N;
- Z: ist bei jedem Auftreten CR¹;
- R¹, R², R³: gleich oder verschieden bei jedem Auftreten H, eine geradkettige, verzweigte oder cyclische Alkylgruppe mit 1 bis 40 C-Atomen, bevorzugt ohne H-Atome in α-Position zur Ketobzw. Iminfunktion, wobei ein oder mehrere nicht benachbarte CH₂-Gruppen durch -R⁴C=CR⁴-, -C≡C-, C=O, C=S, C=Se, C=NR⁴, -O-, -S-, -NR⁵- oder -CONR⁶- ersetzt sein können und wobei ein oder mehrere H-Atome durch F, Cl, Br, I ersetzt sein können, oder
ein aromatisches oder heteroaromatisches System mit 1 bis 40 C-Atomen, wobei ein oder mehrere H-Atome durch F, Cl, Br, I ersetzt sein können und die durch einen oder mehrere, nicht aromatische Reste R¹ substituiert sein kann, wobei mehrere Substituenten R¹ und/oder R¹, R² sowohl am selben Ring als auch an den beiden unterschiedlichen Ringen zusammen wiederum ein weiteres mono- oder polycyclisches, aliphatisches oder aromatisches Ringsystem aufspannen können;
- R⁴, R⁵, R⁶: sind wie unter Formel (1) bis (3) beschrieben.

Ebenfalls bevorzugt sind Mischungen, die als Matrixmaterial A mindestens eine Verbindung der Formel (10) bis (15) enthalten, 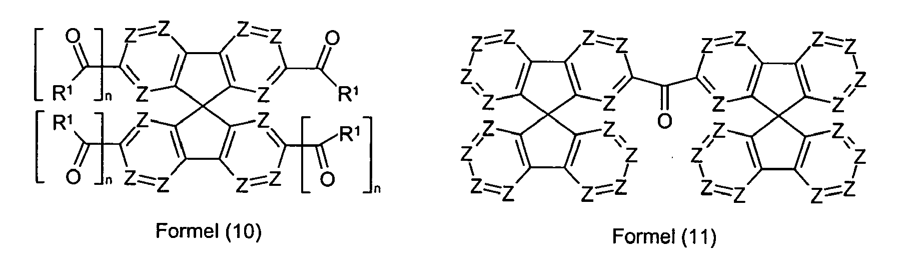 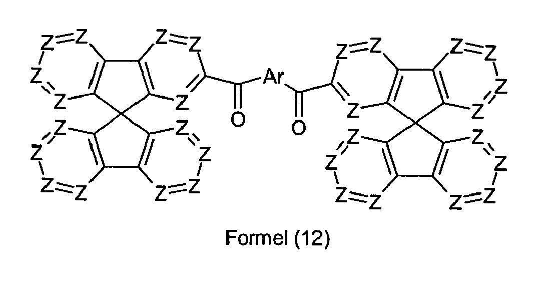 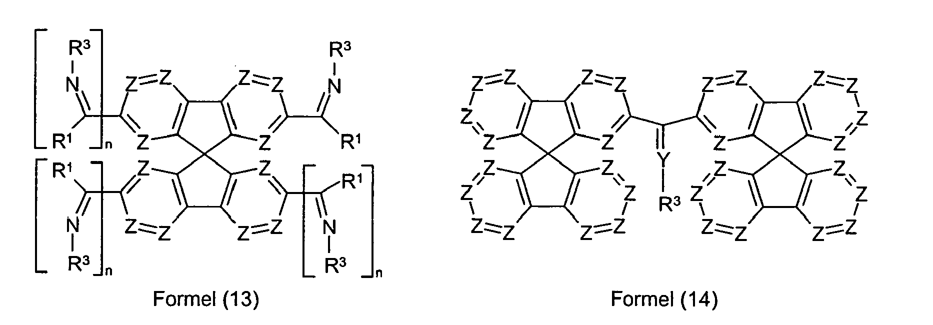 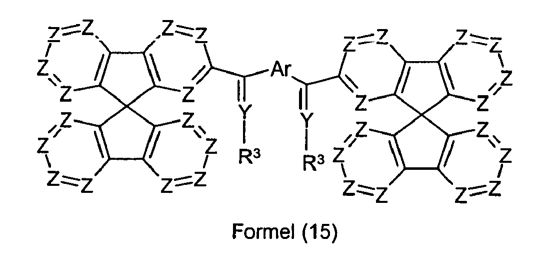 wobei die Symbole Z, Y und R¹ bis R⁶ dieselbe Bedeutung haben, wie unter Formel (1) bis (9) beschrieben, und für die weiteren Symbole und Indizes gilt:
- Ar: ist bei jedem Auftreten gleich oder verschieden ein aromatisches oder heteroaromatisches System mit 2 bis 40 C-Atomen, vorzugsweise mit 4 bis 30 C-Atomen, wobei ein oder mehrere H-Atome durch F, Cl, Br, I ersetzt sein können und die durch einen oder mehrere, nicht aromatische Reste R¹ substituiert sein kann, wobei mehrere Substituenten R¹, sowohl am selben Ring als auch an unterschiedlichen Ringen zusammen wiederum ein weiteres mono- oder polycyclisches, aliphatisches oder aromatisches Ringsystem aufspannen können;
- n: ist bei jedem Auftreten gleich oder verschieden 0 oder 1.

Die Bevorzugung dieser Materialien gemäß Formel (10) bis (15) ist insbesondere durch ihre hohen Glasübergangstemperaturen begründet. Diese liegen je nach Substitutionsmuster typischerweise über 70 °C und meist oberhalb von 100 °C.

Gegenstand der vorliegenden Erfindung sind ebenfalls die neuen Verbindungen gemäß Formel (10a) bis (15), 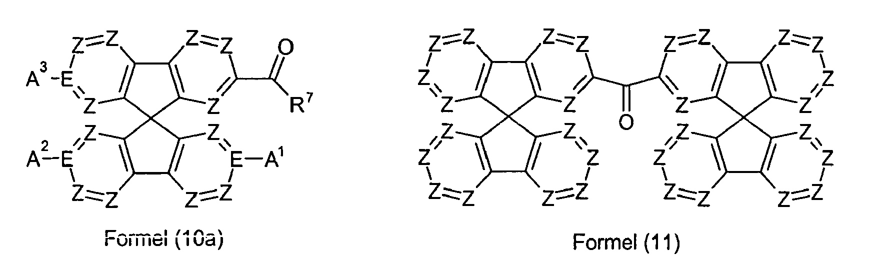 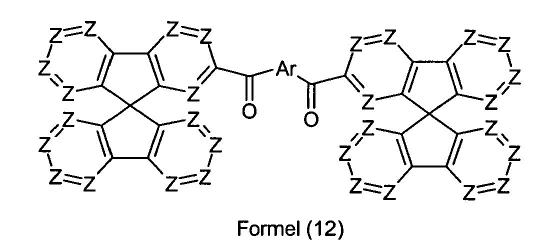 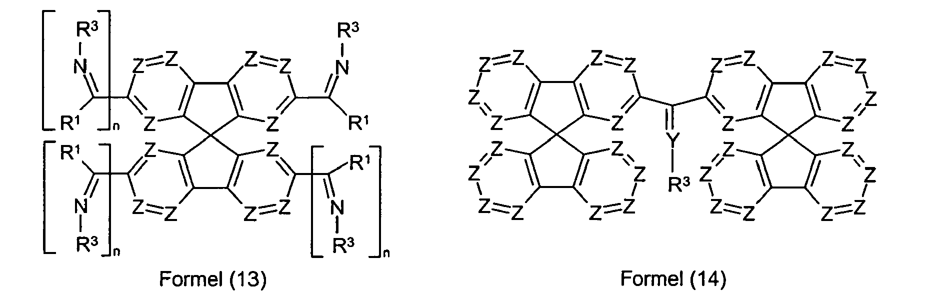 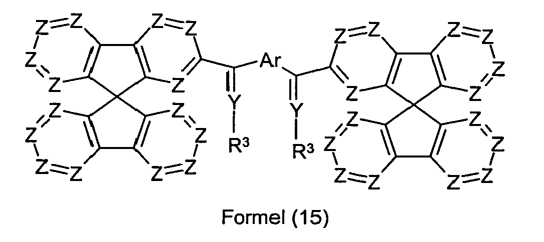 bei denen die Symbole Z, Y, Ar und R¹ bis R⁶ dieselbe Bedeutung haben, wie oben beschrieben, und für die weiteren verwendeten Symbole gilt:
- E: ist bei jedem Auftreten gleich oder verschieden C oder N;
- R⁷: ist bei jedem Auftreten gleich oder verschieden eine Alkyl-, Alkoxy-oder Alkylaminogruppe mit 1 bis 40 C-Atomen, in der auch ein oder mehrere CH₂-Gruppen durch -R⁴C=CR⁴-, -C≡C-, C=O, C=S, C=Se, C=NR⁴, -O-, -S-, -NR⁴- oder -CONR⁴- ersetzt sein können und in der auch ein oder mehrere H-Atome durch F, Cl, Br, I ersetzt sein können, mit der Maßgabe, dass in α-Position zur Carbonylgruppe keine H-Atome gebunden sind, oder
eine aromatische Gruppe, die gegebenenfalls mit Halogen, Alkyl, Trifluormethyl, Hydroxy, -SH, -S-Alkyl, Alkoxy, Nitro, Cyano, -COOH, -CO0Alkyl, -NH₂, -NAlkyl, Benzyl oder Benzoyl substituiert sein kann; oder ein größeres aromatisches System mit 2 bis 40 C-Atomen, vorzugsweise 4 bis 30 C-Atomen, wie beispielsweise 9,9'-Spiro-bifluoren, Fluoren, Triarylamin, etc., wobei ein oder mehrere H-Atome durch F, Cl, Br, I ersetzt sein können und die durch ein oder mehrere nicht-aromatische Reste R¹ substituiert sein kann, wobei mehrere Substituenten R¹ wiederum ein weiteres mono- oder polycyclisches, aliphatisches oder aromatisches Ringsystem aufspannen können;
- A¹: ist bei jedem Auftreten R⁸ oder CO-R⁷, wenn X = C ist, oder ein freies Elektronenpaar, wenn X = N ist;
- A²: ist bei jedem Auftreten R⁸ oder CO-R⁷, wenn X = C ist, oder ein freies Elektronenpaar, wenn X = N ist;
- A³: ist bei jedem Auftreten R⁸ oder CO-R⁷, wenn X = C ist, oder ein freies Elektronenpaar, wenn X = N ist;
- R⁸: ist gleich oder verschieden bei jedem Auftreten H, F, Cl, Br, I, CN, NO₂, eine geradkettige oder verzweigte oder cyclische Alkylgruppe mit 1 bis 40 C-Atomen, wobei ein oder mehrere nicht benachbarte CH₂-Gruppen durch -R⁴C=CR⁴-, -C≡C-, C=S, C=Se, C=NR⁴, -O-, -S-, -NR⁴- oder ―CONR⁴- ersetzt sein können und wobei ein oder mehrere H-Atome durch F, Cl, Br, I ersetzt sein können, oder
ein aromatisches oder heteroaromatisches System mit 1 bis 40 C-Atomen, wobei ein oder mehrere H-Atome durch F, Cl, Br, I ersetzt sein können und die durch einen oder mehrere, nicht-aromatische Reste R¹ substituiert sein kann, wobei mehrere Substituenten R¹ und/oder R¹/R⁴, sowohl am selben Ring als auch an den unter-schiedlichen Ringen zusammen wiederum ein weiteres mono- oder polycyclisches, aliphatisches oder aromatisches Ringsystem aufspannen können;
mit der Maßgabe, dass für die Verbindung gemäß Formel (10a) für die beschriebenen Symbole nur folgende Kombinationen zugelassen sind, wobei R⁸ und R⁴ beliebig gemäß der Definition zu wählen sind:
• wenn R⁷ eine Alkylgruppe ohne α-H-Atome ist, sind die Symbole Z, E, A¹, A² und A³ beliebig gemäß der Definition zu wählen;
• wenn R⁷ eine aromatische Gruppe ist und mindestens ein Z für N steht, sind die Symbole E, A¹, A² und A³ beliebig gemäß der Definition zu wählen;
• wenn R⁷ eine aromatische Gruppe ist und mindestens ein Z für eine Gruppe CR¹ mit R¹ ungleich H steht, sind die Symbole E, A¹, A² und A³ beliebig gemäß der Definition zu wählen;
• wenn R⁷ eine aromatische Gruppe ist und alle Z für CH stehen und mindestens ein Symbol E für N steht, sind die Symbole A¹, A² und A³ beliebig gemäß der Definition zu wählen;
• wenn R⁷ eine aromatische Gruppe ist, alle Z für CH stehen und alle E für C stehen, muss mindestens eines der Symbole A¹, A² und/oder A³ für eine Gruppe R⁸ ungleich Alkyl stehen, während die beiden anderen Gruppen beliebig gemäß der Definition gewählt werden können;
• wenn R⁷ eine aromatische Gruppe ist, alle Z für CH stehen, alle E für C stehen und die beiden Symbole A¹ und A² beliebig gemäß der Definition gewählt sind, wobei mindestens eines der beiden Symbole für eine Gruppe ungleich H steht, dann steht das Symbol
   A³ für eine Gruppe CO-R⁷, wobei R⁷ hier beliebig gemäß der Definition zu wählen ist;
• wenn R⁷ ein größeres aromatisches System, wie beispielsweise Fluoren, Spirobifluoren, Triarylamin, etc., ist, dann sind die Symbole Z, E, A¹, A² und A³ beliebig gemäß der Definition zu wählen.

Der Übersichtlichkeit halber, sind die zugelassenen Kombinationen der Symbole R⁷, Z, E, A¹, A² und A³ für Verbindungen gemäß Formel (10a) in Tabelle 1 zusammengefasst.

**Tabelle 1: Mögliche Kombinationen der Symbole R⁷, Z, E, A¹, A² und A³ für Verbindungen gemäß Formel (10a).**

| **R**^{**7**} | **Z** | **E** | **A**^{**1**} | **A**^{**2**} | **A**^{**3**} |
|---|---|---|---|---|---|
| Alkyl ohne α-H | beliebig gemäß Definition | beliebig gemäß Definition | beliebig gemäß Definition | beliebig gemäß Definition | beliebig gemäß Definition |
| aromatische Gruppe | mind. 1 Z = N | beliebig gemäß Definition | beliebig gemäß Definition | beliebig gemäß Definition | beliebig gemäß Definition |
| aromatische Gruppe | mind. 1 Z = CR¹ mit mind. 1 R¹ ungleich H | beliebig gemäß Definition | beliebig gemäß Definition | beliebig gemäß Definition | beliebig gemäß Definition |
| aromatische Gruppe | alle Z = CH | mindestens 1 E = N | beliebig gemäß Definition | beliebig gemäß Definition | beliebig gemäß Definition |
| aromatische Gruppe | alle Z = CH | alle E = C | R⁸ ungleich Alkyl | beliebig gemäß Definition | beliebig gemäß Definition |
| aromatische Gruppe | alle Z = CH | alle E = C | beliebig gemäß Definition | R⁸ ungleich Alkyl | beliebig gemäß Definition |
| aromatische Gruppe | alle Z = CH | alle E = C | beliebig gemäß Definition | beliebig gemäß Definition | R⁸ ungleich Alkyl |
| aromatische Gruppe | alle Z = CH | alle E = C | beliebig gemäß Definition, ungleich H | beliebig gemäß Definition | CO-R⁷ (R⁷ gemäß Definition) |
| aromatische Gruppe | alle Z = CH | alle E = C | beliebig gemäß Definition | beliebig gemäß Definition, ungleich H | CO-R⁷ (R⁷ gemäß Definition) |
| größeres aromatisches System (z. B. Fluoren, Spirobifluoren, ...) | beliebig gemäß Definition | beliebig gemäß Definition | beliebig gemäß Definition | beliebig gemäß Definition | beliebig gemäß Definition |

Bevorzugt sind Verbindungen, in denen mindestens eine Gruppe R⁷ ein größeres aromatisches System, wie beispielsweise Fluoren, Spirobifluoren, Arylamin, etc. beschreibt.

Bevorzugt sind weiterhin Verbindungen, in denen mindestens eine Gruppe R⁷ für eine Alkylgruppe gemäß der oben genannten Definition ohne α-H-Atome steht.

Bevorzugt sind weiterhin Verbindungen, in denen mindestens eines der Symbole A, B und/oder D für ein aromatisches oder heteroaromatisches System steht.

Bevorzugt sind weiterhin Verbindungen, die mehr als eine Spirobifluoren-Einheit enthalten.

Bevorzugt sind weiterhin Verbindungen, in denen mindestens eines der Symbole Z oder E für N steht.

Bevorzugt sind weiterhin Verbindungen, die mehr als eine Keto-Funktion enthalten, also Diketone oder Oligoketone.

Die vorliegende Erfindung wird durch die folgenden Beispiele für Matrixmaterialien A näher erläutert, ohne sie darauf einschränken zu wollen. Der Fachmann kann aus der Beschreibung und den aufgeführten Beispielen ohne erfinderisches Zutun weitere Matrixmaterialien herstellen und diese in erfindungsgemäßen Mischungen verwenden.

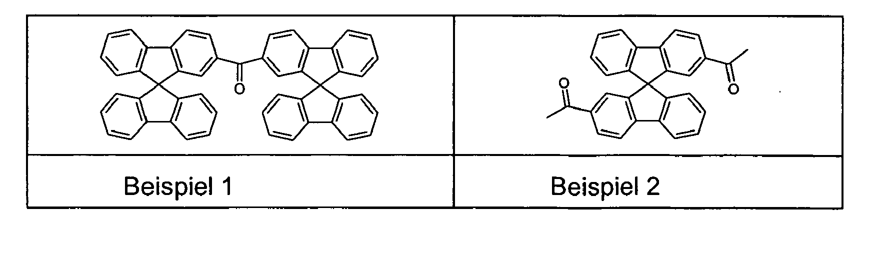

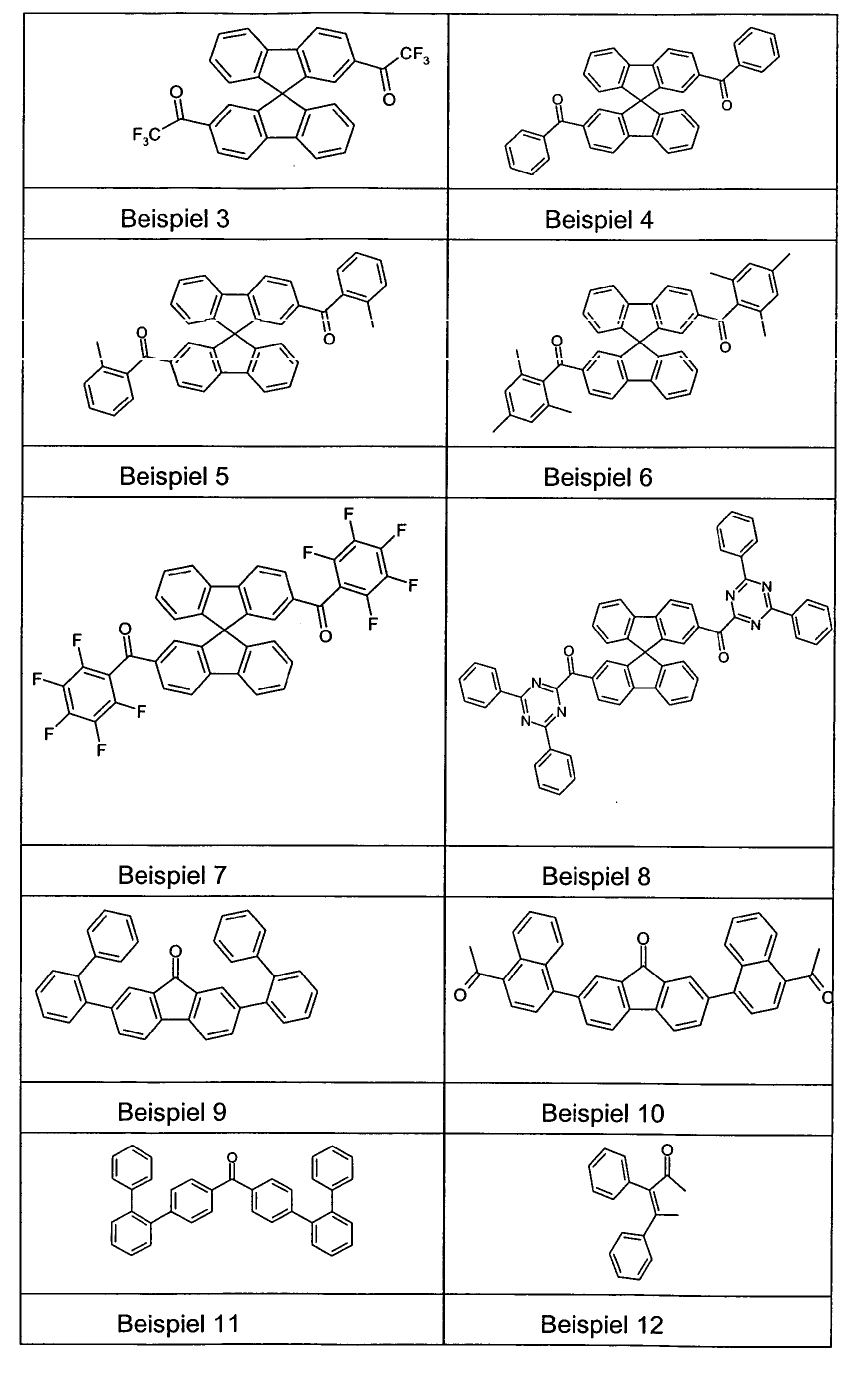

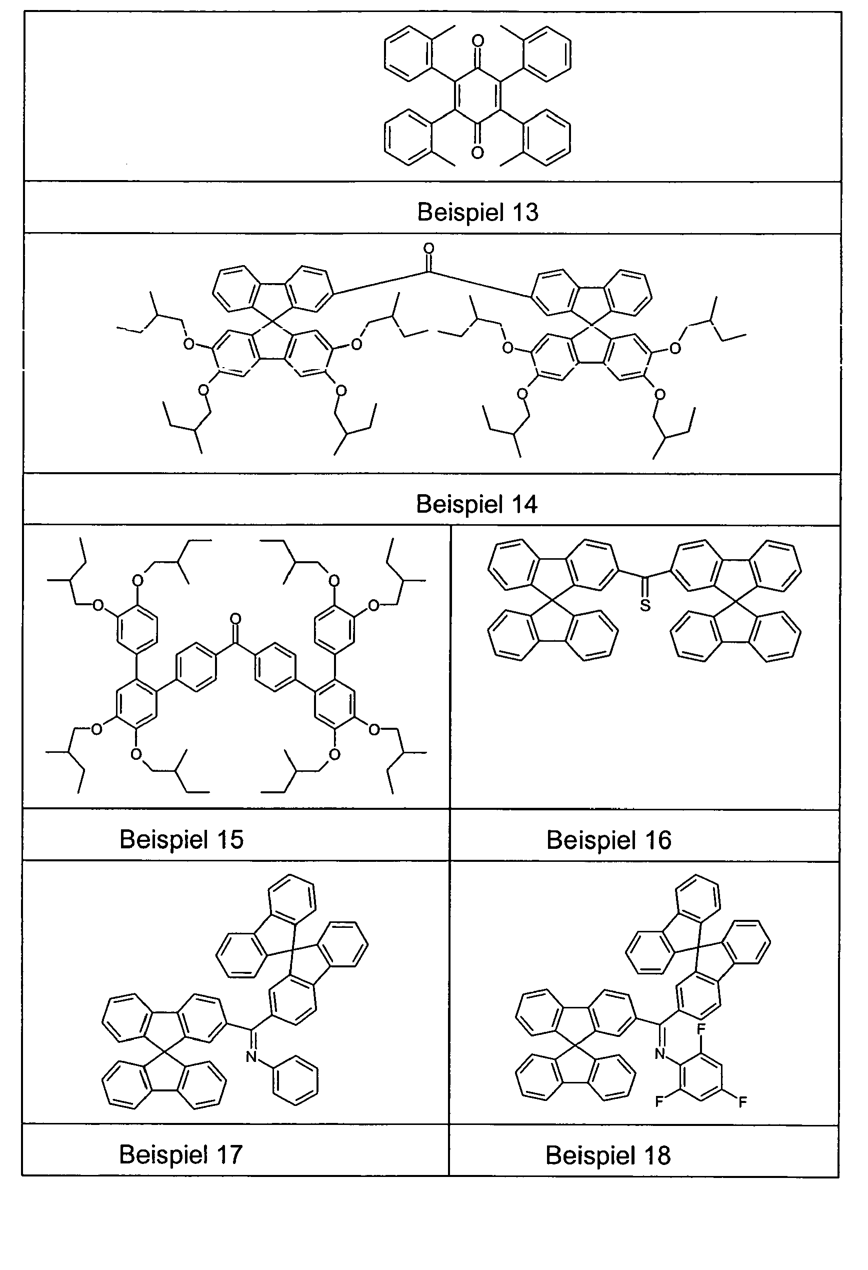

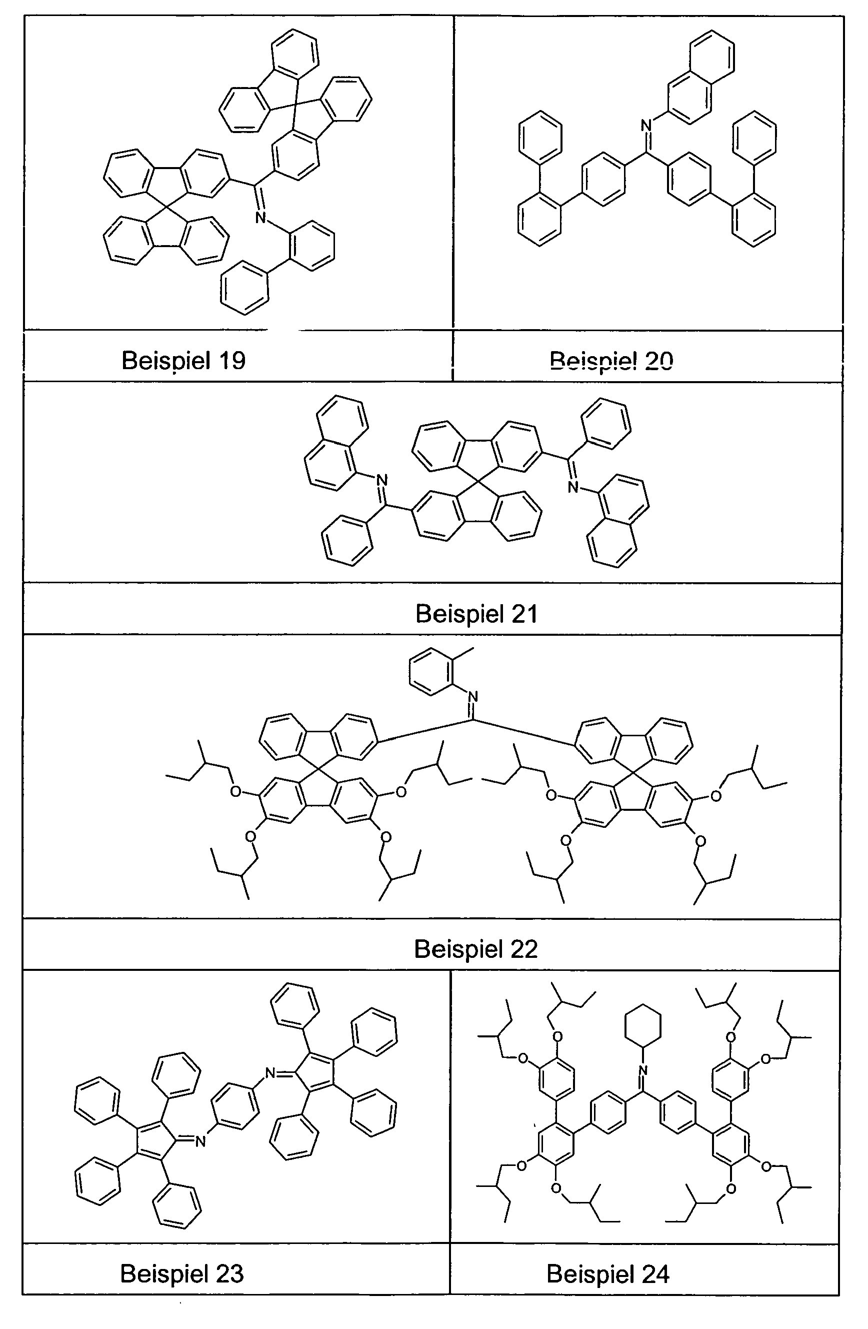

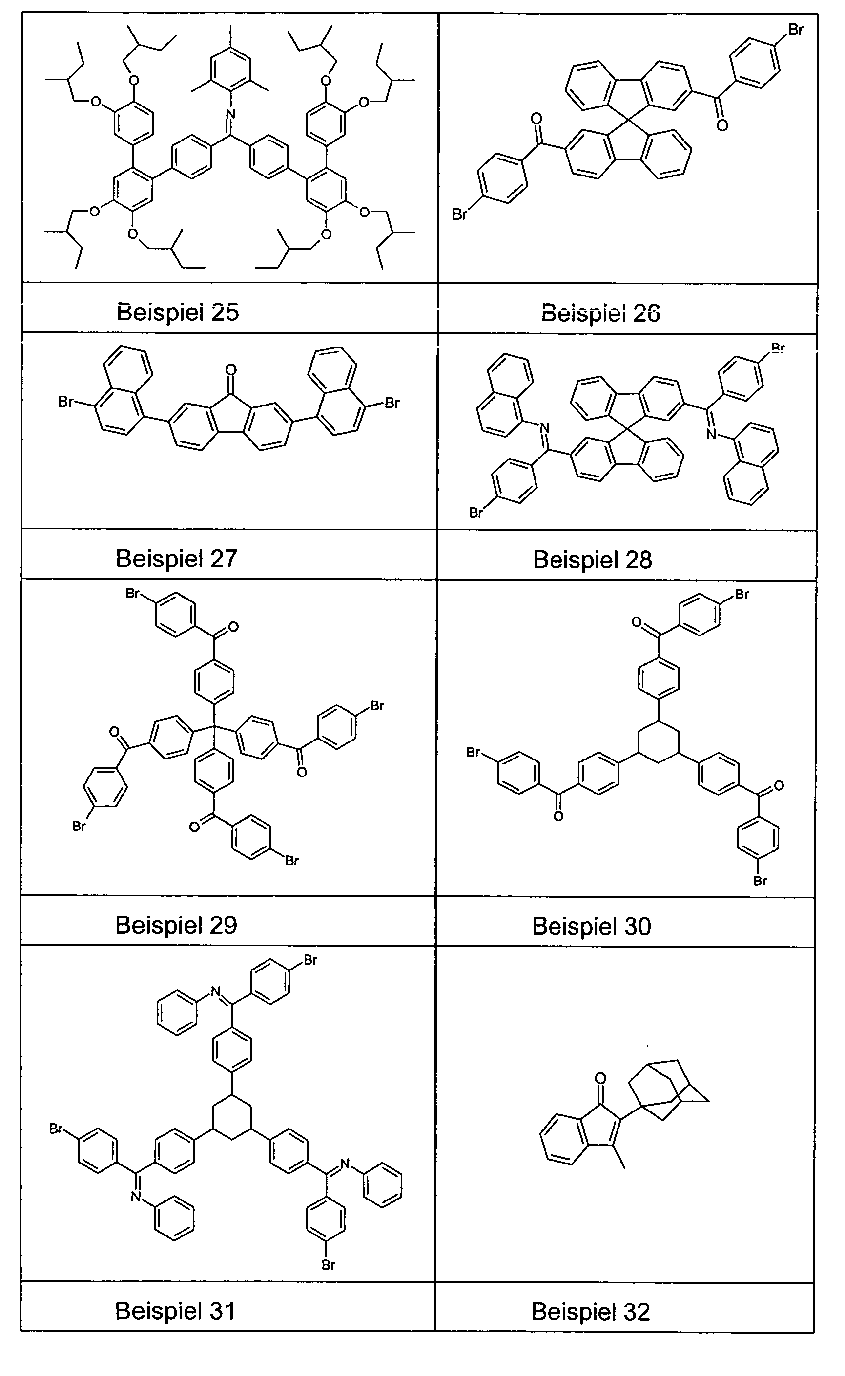

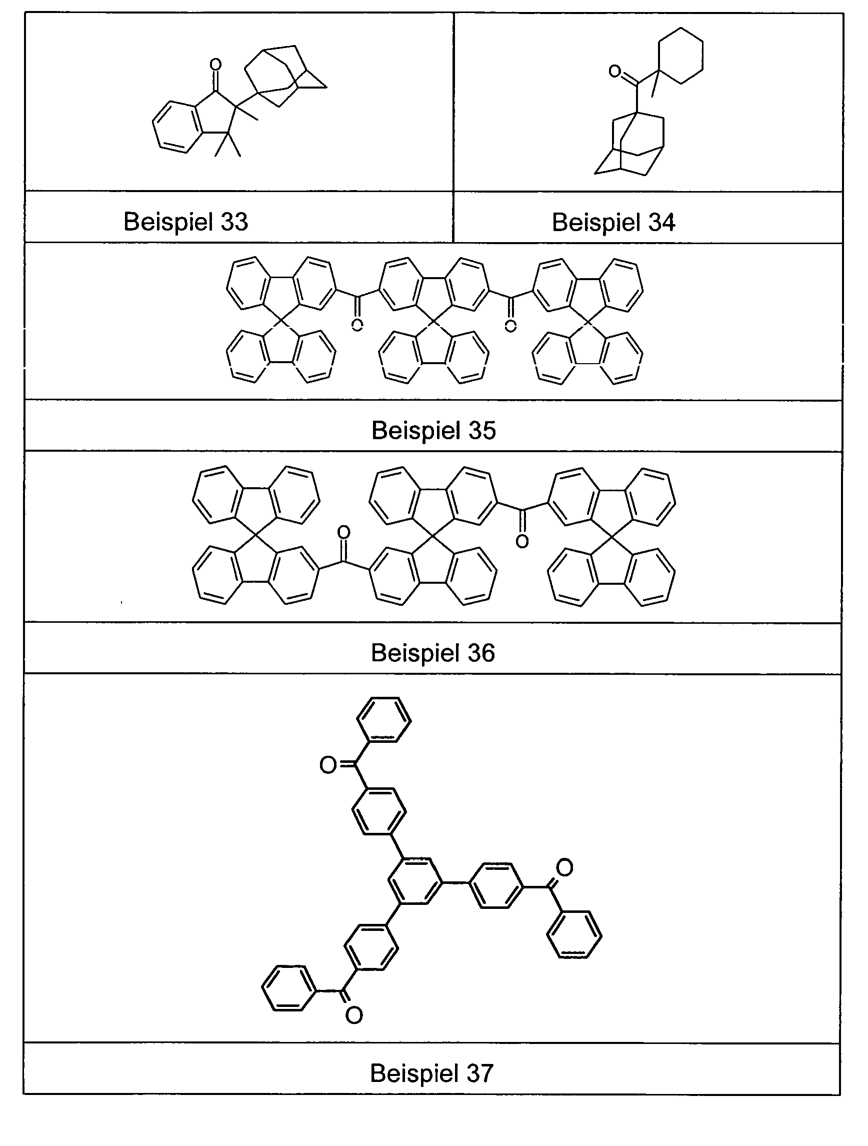

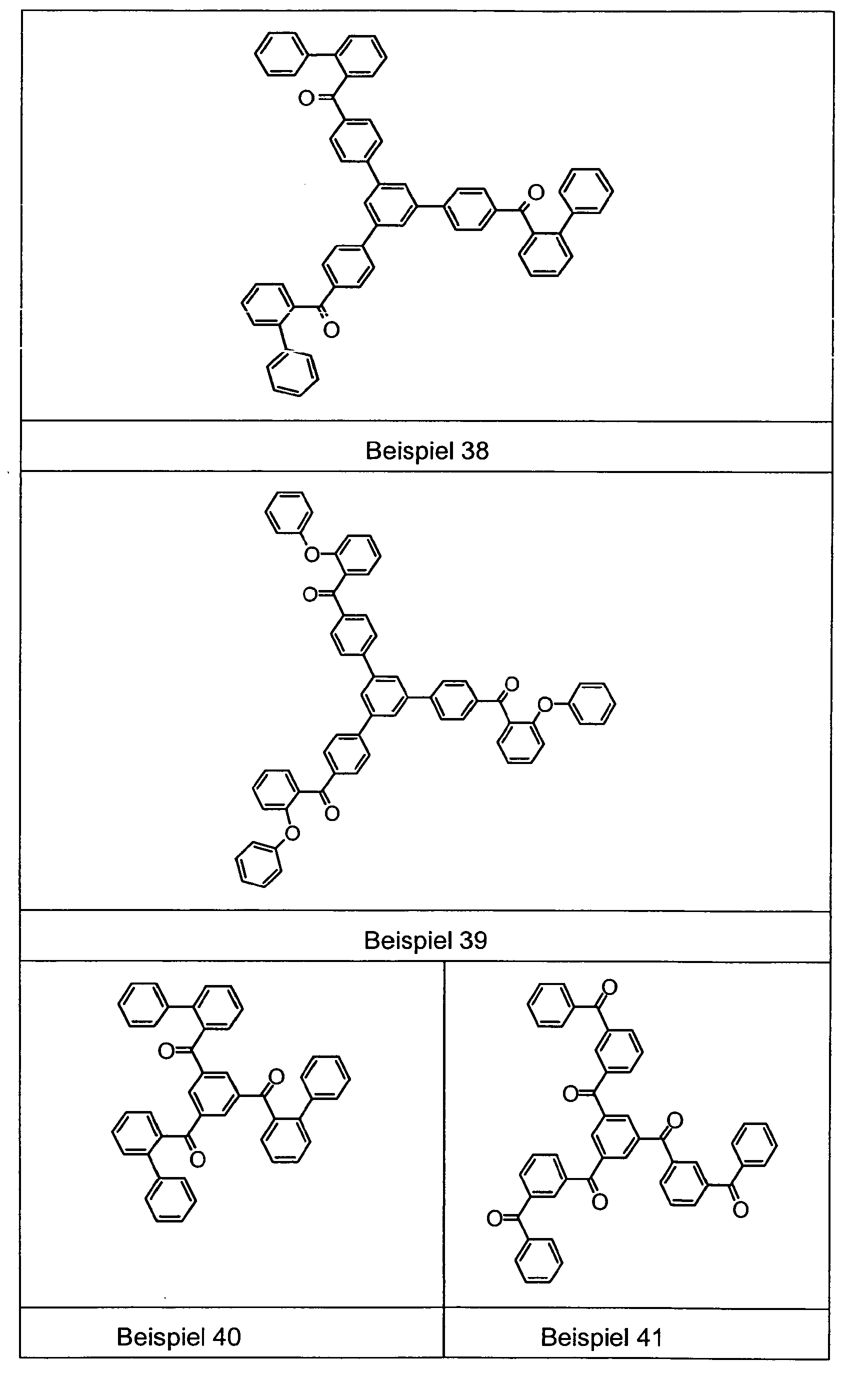

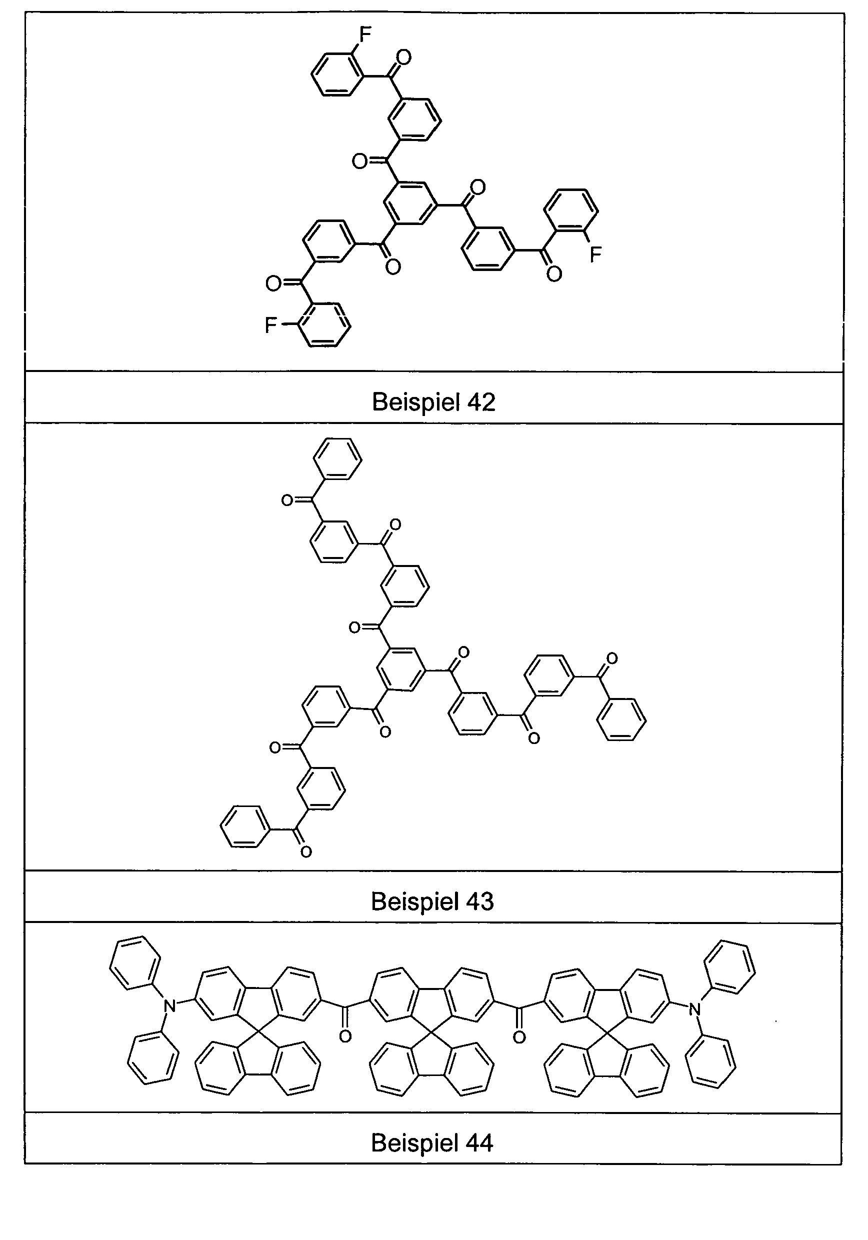

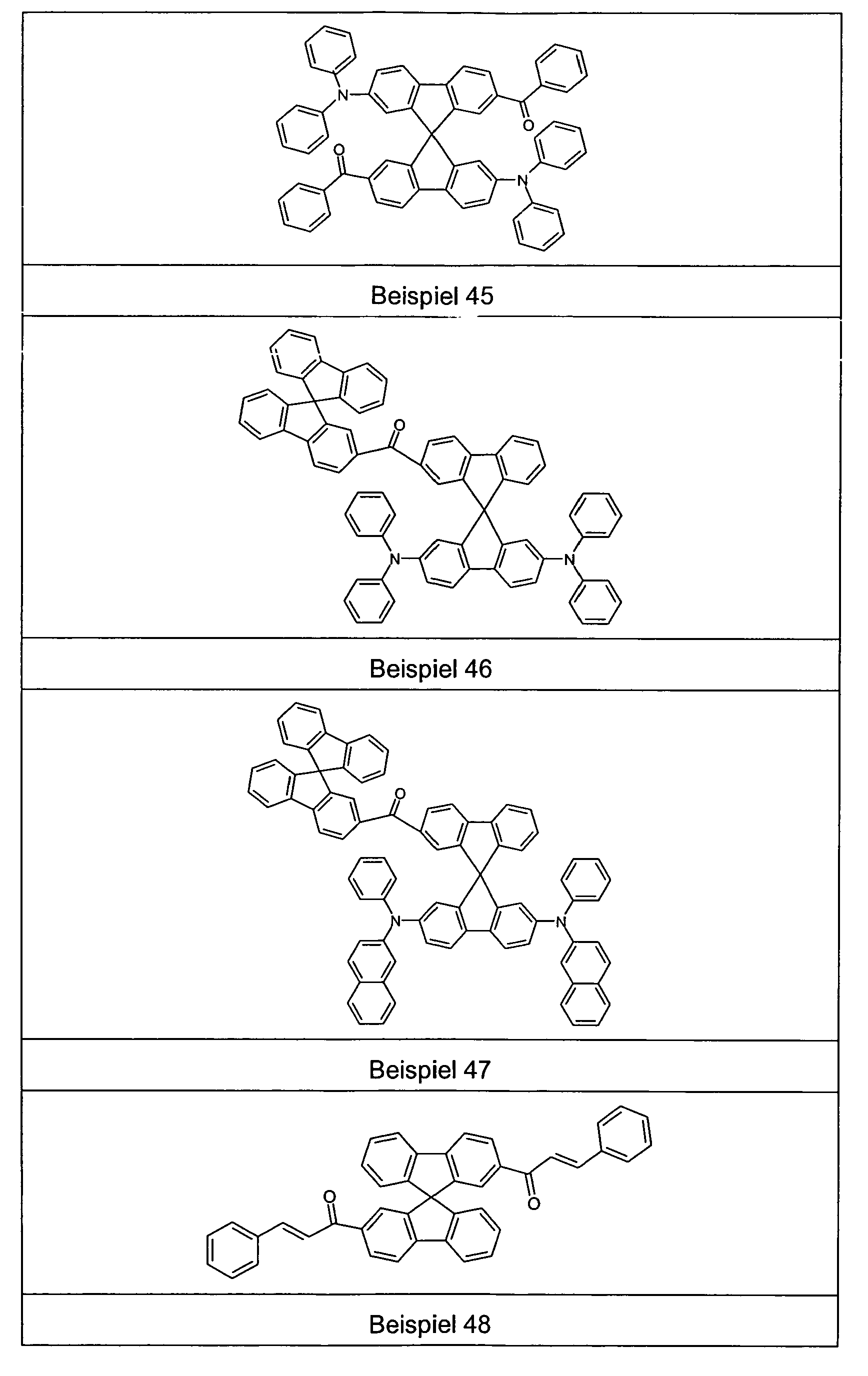

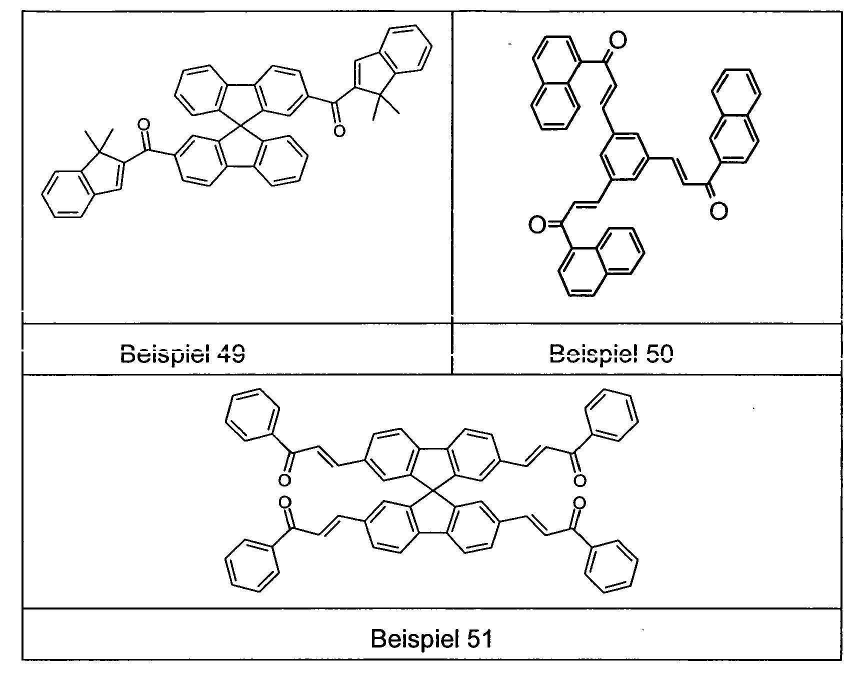

Die oben beschriebenen Matrixmaterialien A ― z. B. gemäß den Beispielen 26, 27 und 28 ― können beispielsweise auch als Co-Monomere zur Erzeugung entsprechender konjugierter, teilkonjugierter oder auch nichtkonjugierter Polymere oder auch als Kern von Dendrimeren ― z. B. gemäß den Beispielen 29, 30 und 31 ― Verwendung finden. Die entsprechende Polymerisation erfolgt dabei bevorzugt über die Halogenfunktionalität.

So können sie u. a. in lösliche Polyfluorene (z. B. gemäß EP 842208 oder WO 00/22026), Poly-spirobifluorene (z. B. gemäß EP 707020 oder EP 894107), Poly-para-phenylene (z. B. gemäß WO 92/18552), Polycarbazole oder auch Polythiophene (z. B. gemäß EP 1028136) einpolymerisiert werden.

Die oben beschriebenen konjugierten, teilkonjugierten oder nichtkonjugierten Polymere oder Dendrimere, die eine oder mehrere Struktureinheiten der Formel (1) bis (15) enthalten, können als Matrixmaterial in organischen Elektrolumineszenzvorrichtungen verwendet werden.

Weiterhin können die erfindungsgemäßen Matrixmaterialien A auch durch die beispielsweise o. g. Reaktionstypen weiter funktionalisiert werden, und so zu erweiterten Matrixmaterialien A umgesetzt werden. Hier sind als Beispiele die Funktionalisierung mit Arylboronsäuren gemäß SUZUKI oder mit Aminen gemäß HARTWIG-BUCHWALD zu nennen.

Um als Funktionsmaterial Verwendung zu finden, werden die erfindungsemäßen Matrixmaterialien A oder deren Mischungen oder die Matrixmaterialien A enthaltende Polymere oder Dendrimere, gegebenenfalls zusammen mit den Emittern B, nach allgemein bekannten, dem Fachmann geläufigen Methoden, wie Vakuumverdampfung, Verdampfen im Trägergasstrom oder auch aus Lösung durch Spincoaten oder mit verschiedenen Druckverfahren (z. B. Tintenstrahldrucken, Off-Set-Drucken, LITI-Druck, etc.) in Form eines Films auf ein Substrat aufgebracht.

Dabei kann die Verwendung von Druckverfahren Vorteile hinsichtlich der Skalierbarkeit der Fertigung, als auch bezüglich der Einstellung von Mischungsverhältnissen in verwendeten Blend-Schichten haben.

Die oben beschriebenen Matrixmaterialien werden in Kombination mit Phosphoreszenz-Emittern verwendet. Diese Mischungen zeichnen sich dadurch aus, dass sie als Emitter B mindestens eine Verbindung enthalten, die dadurch gekennzeichnet ist, dass sie bei geeigneter Anregung Licht emittiert und außerdem mindestens ein Atom der Ordungszahl größer 20, bevorzugt größer 38 und kleiner 84, besonders bevorzugt größer 56 und kleiner 80 enthält.

Bevorzugt werden als Phosphoreszenz-Emitter in den oben beschriebenen Mischungen Verbindungen, die Molybdän, Wolfram, Rhenium, Ruthenium, Osmium, Rhodium, Iridium, Palladium, Platin, Silber, Gold oder Europium enthalten, verwendet.

Besonders bevorzugte Mischungen enthalten als Emitter B mindestens eine Verbindung der Formel (16) bis (19), 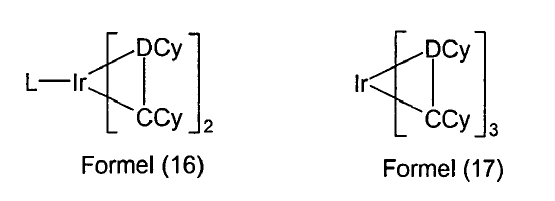 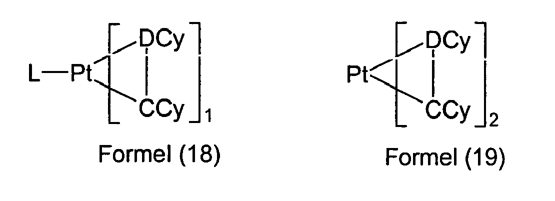 worin für die verwendeten Symbole und Indizes gilt:
- DCy: ist bei jedem Auftreten gleich oder verschieden eine cyclische Gruppe, die mindestens ein Donoratom, bevorzugt Stickstoff oder Phosphor, enthält, über welches die cyclische Gruppe an das Metall gebunden ist, und die wiederum ein oder mehrere Substituenten R⁹ tragen kann; die Gruppen DCy und CCy sind über eine kovalente Bindung miteinander verbunden;
- CCy: ist bei jedem Auftreten gleich oder verschieden eine cyclische Gruppe, die ein Kohlenstoffatom enthält, über welches die cyclischen Gruppe an das Metall gebunden ist, und die wiederum ein oder mehrere Substituenten R⁹ tragen kann;
- R⁹: ist gleich oder verschieden und bei jedem Auftreten H, F, Cl, Br, I, NO₂, CN, eine geradkettige oder verzweigte oder cyclische Alkyl- oder Alkoxygruppe mit 1 bis 40 C-Atomen, wobei ein oder mehrere nicht benachbarte CH₂-Gruppen durch -CR⁴=CR⁴-, -C≡C-, C=O, C=S, C=Se, C=NR⁴, -O-, -S-, -NR⁵- oder-CONR⁶⁻ersetzt sein können und wobei ein oder mehrere H-Atome durch F ersetzt sein können, oder ein aromatisches oder hetero-aromatisches System mit 4 bis 40 C-Atomen, das durch einen oder mehrere, nicht aromatische Reste R⁹ substituiert sein kann, wobei mehrere Substituenten R⁹, sowohl am selben Ring als auch an den beiden unterschiedlichen Ringen zusammen wiederum ein weiteres mono- oder polycyclisches, aliphatisches oder aromatisches Ringsystem aufspannen können;
- L: ist gleich oder verschieden bei jedem Auftreten ein zweizähnig, chelatisierender Ligand, bevorzugt ein Diketonat-Ligand,
- R⁴, R⁵, R⁶: ist gleich oder verschieden bei jedem Auftreten H oder ein aliphatischer oder aromatischer Kohlenwasserstoffrest mit 1 bis 20 C-Atomen.

Beispiele der oben beschriebenen Emitter können zum Beispiel den Anmeldungen WO 00/70655, WO 01/41512, WO 02/02714, WO 02/15645, EP 1191613, EP 1191612, EP 1191614, WO 03/099959, WO 03/084972, WO 03/040160, WO 02/081488, WO 02/068435 und DE 0238903.9 entnommen werden; diese werden hiermit via Zitat als Bestandteil der Anmeldung betrachtet.

Die erfindungsgemäße Mischung enthält zwischen 1 bis 99 Gew.%, vorzugsweise zwischen 3 und 95 Gew.%, besonders bevorzugt zwischen 5 und 50 Gew.%, insbesondere zwischen 7 und 20 Gew.% Emitter B bezogen auf die Gesamtmischung aus Emitter B und Matrixmaterial A.

Weiterer Gegenstand der vorliegenden Erfindung sind elektronische Bauteile, insbesondere organische Elektrolumineszenzvorrichtungen (OLEDs), organische Solarzellen (O-SCs), organische Feldeffekttransistoren (O-FETs) oder auch organische Laserdioden (O-Laser), enthaltend die erfindungsgemäße Mischung aus Matrixmaterial A und Emissionsmaterial B.

Besonders bevorzugt sind organische Elektrolumineszenzvorrichtungen, die eine emittierende Schicht (EML) aufweisen, enthaltend eine erfindungsgemäße Mischung aus mindestens einem Matrixmaterial A und mindestens einem zur Emission befähigten Emissionsmaterial B.

Insbesondere bevorzugt werden organische Elektrolumineszenzvorrichtungen, die in der emittierenden Schicht (EML) mindestens eine erfindungsgemäße Mischung enthalten, wobei die Glastemperatur T_{g} der Reinsubstanz des Matrixmaterials A größer 70 °C ist.

Die organische Elektrolumineszenzvorrichtung kann außer der Kathode, der Anode und der emittierenden Schicht weitere Schichten enthalten, wie z. B. Lochinjektionsschicht, Lochtransportschicht, Lochblockierschicht, Elektronentransportschicht und/oder Elektroneninjektionsschicht. Es sei aber an dieser Stelle darauf hingewiesen, dass nicht notwendigerweise jede dieser Schichten vorhanden sein muss.

So hat sich beispielsweise gezeigt, dass eine OLED, die weder eine separate Lochblockierschicht, noch eine separate Elektronentransportschicht enthält, sehr gute Ergebnisse in der Elektrolumineszenz zeigt, insbesondere eine nochmals deutlich niedrigere Spannung und höhere Leistungseffizienz. Dies ist besonders überraschend, da eine entsprechende OLED mit einem Carbazol-haltigen Matrixmaterial ohne Lochblockier- und Elektronentransportschicht nur sehr geringe Leistungseffizienzen zeigt, insbesondere bei hoher Helligkeit (vgl. Adachi et al., Organic Electronics 2001, 2, 37). Ein weiterer Gegenstand der Erfindung ist also eine organische Elektrolumineszenzvorrichtung, enthaltend eine erfindungsgemäße Mischung, die ohne Verwendung einer Lochblockierschicht direkt an die Elektronentransportschicht grenzt oder die ohne Verwendung einer Lochblockierschicht und einer Elektronentransportschicht direkt an die Elektroneninjektionsschicht oder an die Kathode grenzt.

Ebenso hat sich gezeigt, dass eine OLED, die keine separate Lochinjektionsschicht enthält, sondern nur ein oder mehrere Lochtransportschichten (Triarylaminschichten) direkt auf der Anode, ebenfalls sehr gute Ergebnisse in der Elektrolumineszenz zeigt. Dieser Aufbau ist also auch Gegenstand der vorliegenden Erfindung.

Die erfindungsgemäßen organischen Elektrolumineszenzvorrichtungen zeigen höhere Effizienz, deutlich längere Lebensdauer und, insbesondere ohne Verwendung einer Lochblockier- und Elektronentransportschicht, deutlich niedrigere Betriebsspannungen und höhere Leistungseffizienzen als OLEDs gemäß Stand der Technik, die CBP als Matrixmaterial enthalten. Durch Weglassen der Lochblockier- und Elektronentransportschichten vereinfacht sich weiterhin der Aufbau der OLED deutlich, was einen erheblichen technologischen Vorteil darstellt.

Die bevorzugten Ausführungsformen der erfindungsgemäßen Mischungen aus Matrixmaterial A und Emissionsmaterial B sind auch für die erfindungsgemäßen elektronischen Bauteile, insbesondere für die organischen Elektrolumineszenzvorrichtungen (OLEDs), organischen Solarzellen (O-SCs), organischen Feldeffekttransistoren (O-FETs) oder auch organischen Laserdioden (O-Laser) gegeben. Zur Vermeidung von unnötigen Wiederholungen wird daher auf erneute Aufzählung an dieser Stelle verzichtet.

Im vorliegenden Anmeldetext und auch in den im weiteren folgenden Beispielen wird nur auf organische Leuchtdioden und die entsprechenden Displays abgezielt. Trotz dieser Beschränkung der Beschreibung ist es für den Fachmann ohne weiteres erfinderisches Zutun möglich, entsprechende erfindungsgemäße Schichten aus den erfindungsgemäßen Mischungen herzustellen und anzuwenden, insbesondere in OLED-nahen oder verwandten Anwendungen.

### Beispiele

### 1. Synthese von Matrixmaterialien A:

Die nachfolgenden Synthesen wurden - sofern nicht anders angegeben - unter einer Schutzgasatmosphäre in getrockneten Lösungsmitteln durchgeführt. Die Edukte wurden von ALDRICH [Kupfer(I)cyanid, Acetylchlorid, N-Methylpyrrolidinon (NMP)] bezogen. 2-Brom-9,9'-spiro-bifluoren, 2,7-Dibrom-9,9'-spiro-bifluoren (J. Pei et al., J. Org. Chem. 2002, 67(14), 4924-4936) und 9,9'-Spirobifluoren-2,2'-dicarbonsäurechlorid (V. A. Montero et al., Tetrahedron Lett. 1991, 32(39), 5309-5312) wurden nach Literaturmethoden dargestellt.

### Beispiel 1 : Bis(9,9'-spiro-bifluoren-2-yl)keton

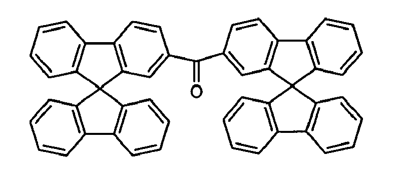

### A: 2-Cyano-9,9'-spiro-bifluoren

Eine Suspension von 158.1 g (0.4 mol) 2-Brom-9,9'-spiro-bifluoren und 89.6 g (1 mol) Kupfer(I)cyanid in 1100 ml NMP wurde 16 h auf 160 °C erhitzt. Nach Abkühlen auf 30 °C wurde mit 1000 ml gesättigter Ammoniak-Lösung versetzt und 30 min. nachgerührt. Der Niederschlag wurde abgesaugt, dreimal mit 300 ml gesättigter Ammoniak-Lösung und dreimal mit 300 ml Wasser gewaschen und trocken gesaugt. Nach Lösen des Feststoffs in 1000 ml Dichlormethan wurde die Lösung über Natriumsulfat getrocknet, über Kieselgel abfiltriert und zur Trockene eingeengt. Das so erhaltene Rohprodukt wurde einmal aus Dioxan : Ethanol (400 ml : 750 ml) umkristallisiert. Nach Trocknen der Kristalle im Vakuum bei 80 °C wurden 81.0 g (237 mmol) entsprechend 59.3 % der Th. erhalten.
¹H-NMR (CDCl₃): δ [ppm] = 7.92 - 7.85 (m, 4 H), 7.66 - 7.65 (m, 1 H), 7.44 - 7.39 (m, 3 H), 7.22 - 7.19 (m, 1 H), 7.15 - 7.11 (m, 2 H), 6.99 - 6.98 (m, 1 H), 6.79 - 6.78 (m, 1 H), 6.69 - 6.67 (m, 2 H).

### B: Bis(9,9'-spiro-bifluoren-2-yl)keton

Aus einer Lösung von 98.8 g (250 mmol) 2-Brom-9,9'-spiro-bifluoren und 6 ml 1,2-Dichlorethan in 1000 ml THF und 7.1 g (290 mmol) Magnesium wurde in der Siedehitze das entsprechende Grignard-Reagens hergestellt. Zu dieser Grignard-Lösung wurde bei 0-5 °C eine Lösung von 85.4 g (250 mmol) 2-Cyano-9,9'-spiro-bifluoren in einer Mischung aus 300 ml THF und 1000 ml Toluol während 15 min. zugetropft. Anschließend wurde die Mischung 6 h unter Rückfluss erhitzt. Nach Abkühlen wurde eine Mischung von 35 ml 10N HCl, 400 ml Wasser und 600 ml Ethanol langsam zugetropft. Nach 16 h Rühren bei Raumtemperatur wurde der Feststoff abgesaugt und dreimal mit 200 ml Ethanol gewaschen. Der Feststoff wurde viermal aus NMP (5 ml/g) umkristallisiert und anschließend im Hochvakuum (T = 385 °C, p = 5 x 10⁻⁵ mbar) sublimiert. Die Ausbeute bei einer Reinheit > 99.9 % nach HPLC betrug 52.1 g (79 mmol) entsprechend 31.6 % der Th.
T₉ = 165 °C, Tₘ = 385 °C.
¹H-NMR (CDCl₃): δ [ppm] = 7.87 - 7.85 (m, 2H), 7.83 - 7.81 (m, 4H), 7.78 - 7.86 (m, 2H), 7.60 - 7.58 (m, 2H), 7.39 - 7.34 (m, 6H), 7.18 - 7.17 (m, 2H), 7.16 - 7.13 (m, 2H), 7.10 - 7.07 (m, 4H), 6.34 - 6.32 (m, 2H), 6.70-6.69 (m, 4H).

### Beispiel 2: 2,2'-Bis(benzoyl)-spiro-9,9'-bifluoren

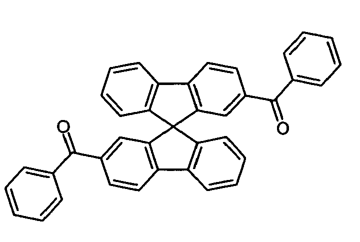

Eine Suspension von 160.0 g (1.2 mol) wasserfreiem Aluminiumchlorid in 600 ml 1,2-Dichlorethan wurde tropfenweise unter gutem Rühren mit 132 ml (1.1 mol) Benzoylchlorid versetzt. Zu dieser Mischung wurde eine Lösung von 158.2 g (0.5 mol) Spiro-9,9'-bifluoren in 600 ml 1,2-Dichlorethan so zugetropft, dass die Temperatur 25 °C nicht überschritt. Nach vollendeter Zugabe wurde noch 1 h bei Raumtemperatur gerührt. Anschließend goss man die Reaktionsmischung auf ein gut gerührtes Gemisch aus 1000 g Eis und 260 ml 2N Salzsäure. Die organische Phase wurde abgetrennt und zweimal mit 500 ml Wasser gewaschen. Nach Einengen der organischen Phase auf ein Volumen von ca. 200 ml und Zugabe von 500 ml Ethanol wurde der gebildete feinkristalline Niederschlag abgesaugt und mit Ethanol gewaschen. Der Feststoff wurde wiederholt aus Toluol umkristallisiert und anschließend im Hochvakuum (T = 290 °C, p = 5 x 10⁻⁵ mbar) sublimiert. Die Ausbeute bei einer Reinheit > 99.9 % nach HPLC betrug 191.5 g (365 mmol) entsprechend 73.0 % der Th.
T_{g} = 99 °C, Tₘ = 281 °C.
¹H-NMR (CDCl₃): δ [ppm] = 7.90 (m, 4H), 7.78 (m, 2H), 7.67 (m, 4H), 7.51 (m, 2H), 7.43-7.37 (m, 6H), 7.31 (m, 2H), 7.20 (m, 2H), 6.78 (m, 2H).

### Beispiel 3: 2,2'-Bis(2-fluorbenzoyl)-spiro-9,9'-bifluoren

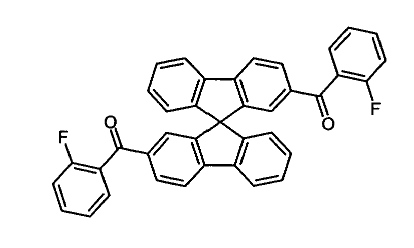

Durchführung analog Beispiel 2. Einsatz von 174.4 g (1.1 mol) 2-Fluorbenzoylchlorid. Der Feststoff wurde wiederholt aus Butanon und Toluol umkristallisiert und anschließend im Hochvakuum (T = 250 °C, p = 5 x 10⁻⁵ mbar) sublimiert. Die Ausbeute bei einer Reinheit > 99.9 % nach HPLC betrug 192.8 g (344 mmol) entsprechend 68.8 % der Th..
T₉ = 96 °C, Tₘ = 228 °C.
¹H-NMR (CDCl₃): δ [ppm] = 7.90 (m, 4H), 7.77 (m, 2H), 7.48 - 7.40 (m, 6H), 7.37 (m, 2H), 7.21-7.18 (m, 4H), 7.09 (m, 2H), 6.77 (m, 2H).
¹⁹F-{¹H}-NMR (CDCl₃): δ [ppm] = -111.7 (s).

### Beispiel 4: 2,7-Bis(2-Spiro-9,9'-bifluorenyl-carbonyl)-spiro-9,9'-bifluoren

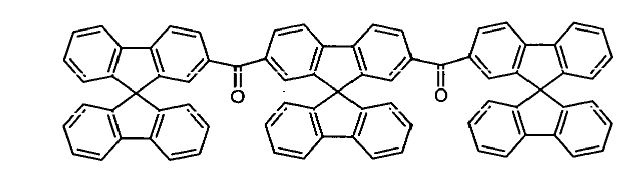

Durchführung analog Beispiel 1B. Einsatz von 59.3 g (125 mmol) 2,7-Dibrom-spiro-9,9'-bifluoren. Sublimation bei T = 410 °C. Ausbeute 77.1 g (77 mmol), entsprechend 61.6 % d. Th..
T_{g} = 209 °C, Tₘ = 401 °C.
¹H-NMR (CDCl₃): δ [ppm] = 7.87-7.75 (m, 12H), 7.61-7.56 (m, 4H), 7.40-7.34 (m, 8H), 7.18-7.14 (m, 6H), 7.11-7.07 (m, 6H), 6.74-6.67 (m, 8H).

### Beispiel 5: 2,2'-Bis(2-spiro-9,9'-bifluorenylcarbonyl)-spiro-9,9'-bifluoren

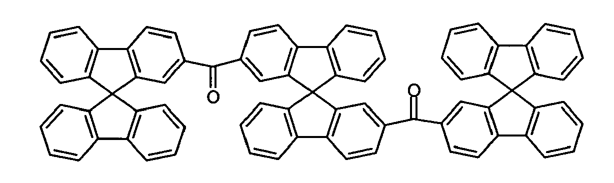

### A: 9,9'-Spirobifluoren-2,2'-dicarbonsäureamid

220 ml einer Ammoniaklösung (2N in Ethanol) wurden unter gutem Rühren mit 44.1 g (100 mmol) 9,9'-Spirobifluoren-2,2'-dicarbonsäurechlorid, gelöst in 200 ml Dioxan, tropfenweise versetzt. Nach Abklingen der exothermen Reaktion wurde noch 2 h nachgerührt, der ausgefallene Feststoff wurde abfiltriert, einmal mit einer Mischung aus 100 ml Wasser und 100 ml EtOH und einmal mit 200 ml Ethanol gewaschen und im Vakuum getrocknet. Die Ausbeute bei einer Reinheit > 99.0 % nach ¹H-NMR betrug 37.4 g (93 mmol) entsprechend 93.0 % der Th.
¹H-NMR (DMSO-d6): δ [ppm] = 8.13-8.10 (m, 4H), 8.01-7.99 (m, 2H), 7.89 (br. s, 2H, NH₂), 7.47-7.44 (m, 2H), 7.23 (br. s, 2H, NH₂), 7.22-7.18 (m, 2H), 7.14 (s, 2H), 6.66-6.64 (m, 2H).

### B: 2,2'-Dicyano-spiro-9,9'-bifluoren

Eine auf -10 °C gekühlte Suspension von 36.2 g (90 mmol) 9,9'-Spirobifluoren-2,2'-dicarbonsäureamid in 800 ml DMF wurde tropfenweise so mit 52.5 ml (720 mmol) Thionylchlorid versetzt, dass die Temperatur nicht über -5 °C anstieg. Die Reaktionsmischung wurde weitere 3 h bei -10 °C gerührt und dann in ein Gemisch aus 2 kg Eis und 500 ml Wasser gegossen. Das Hydrolysat wurde zweimal mit je 500 ml Dichlormethan extrahiert. Die vereinigten organischen Phasen wurde mit 500 ml Wasser und mit 500 ml ges. Kochsalzlösung gewaschen und über Magnesiumsulfat getrocknet. Das nach Einengen der organischen Phase erhaltene Öl kristallisierte nach Zugabe von 300 ml Ethanol in Form von weißen Nadeln. Die Ausbeute bei einer Reinheit > 99.0 % nach ¹H-NMR betrug 29.4 g (80 mmol) entsprechend 89.3 % der Th.
¹H-NMR (CDCl₂CDCl₂): δ [ppm] = 7.95 (d, 2H), 7.92 (d, 2H), 7.71 (dd, 2H), 7.47 (ddd, 2H), 7.24 (ddd, 2H), 6.96 (d, 2H), 6.75 (d, 2H).

### C: 2,2'-Bis(2-spiro-9,9'-bifluorenyl-carbonyl)-spiro-9,9'-bifluoren

Durchführung analog Beispiel 1 B. Einsatz von 59.3 g (150 mmol) 2-Brom-9,9'-spiro-bifluoren und 27.5 g (75 mmol) 2,2'-Dicyano-spiro-9,9'-bifluoren. Sublimation bei T = 440 °C. Ausbeute 41.2 g (41 mmol), entsprechend 54.8 % d. Th..
T_{g} = 213 °C, Tₘ = 430 °C.
¹H-NMR (CDCl₃): δ [ppm] = 7.89-7.86 (m, 4H), 7.82-7.78 (m, 8H), 7.60 (br. m, 4H), 7.41-7.34 (m, 8H), 7.18-7.14 (m, 8H), 7.12-7.08 (4H), 6.75-6.70 (m, 8H).

### Beispiel 6: Bis(9,9'-spiro-bifluoren-2-yl)-N-tert-butylimin

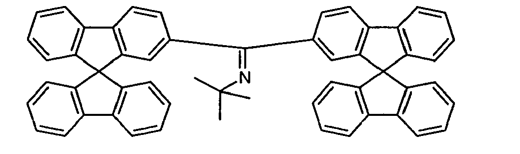

Zu einer auf 0 °C gekühlten Suspension von 65.8 g (100 mmol) Bis(9,9'-spiro-bifluoren-2-yl)keton (Darstellung s. Beispiel 1) in einem Gemisch aus 105.0 ml (1 mol) Tert-butylamin und 1500 ml Toluol wurden 200 ml (200 mmol) einer 2M Lösung von Titantetrachlorid in Toluol während 30 min. zugetropft. Anschließend wurde das Kältebad entfernt, die Reaktionsmischung wurde nach Erreichen der Raumtemperatur noch 3 h nachgerührt und dann 60 h unter Rückfluß erhitzt. Nach Erkalten wurden 1500 ml Diethylether zugesetzt und weitere 12 h bei Raumtemperatur gerührt. Die Suspension wurde über Kieselgel filtriert, das Filtrat wurde zur Trockene eingeengt, in 2000 ml Chloroform aufgenommen und erneut über Kieselgel filtriert. Der nach Entfernen des Chloroforms verbliebene Feststoff wurde viermal aus Dioxan / Ethanol (1:2 vv, 10 ml/g) umkristallisiert und anschließend im Hochvakuum (T = 375 °C, p = 5 x 10⁻⁵ mbar) sublimiert. Die Ausbeute bei einer Reinheit > 99.9 % nach HPLC betrug 47.8 g (67 mmol) entsprechend 67.0 % der Th.
T₉ = 187 °C, Tₘ = 369 °C.
¹H-NMR (CDCl₃): δ [ppm] = 7.89 - 7.72 (m, 7H), 7.62 (d, 1 H), 7.37 - 7.26 (m, 7H), 7.11 - 7.01 (m, 7H), 6.98 (s, 1 H), 6.71 (d, 1H), 6.64 - 6.59 (m, 5H), 6.44 (s, 1H), 0.83 (s, 9H).

### Beispiel 7: Bis(9,9'-spiro-bifluoren-2-yl)-N-phenylimin

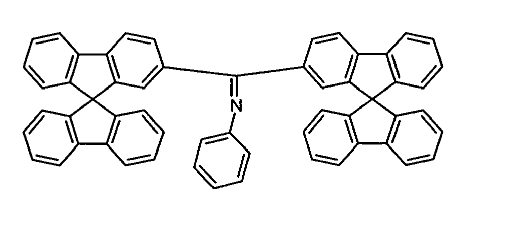

Durchführung analog Beispiel 6. Einsatz von 45.6 ml (500 mmol) Anilin. Sublimation bei T = 370 °C. Ausbeute 53.7 g (73 mmol), entsprechend 73.2 % d. Th.
T₉ = 159 °C, Tₘ = 339 °C.
¹H-NMR (CDCl₃): δ [ppm] = 7.82 - 7.74 (m, 6H), 7.70 (d, 1 H), 7.65 (d, 1 H), 7.44 (s, 1 H), 7.38 - 7.29 (m, 7H), 7.12 - 7.02 (m, 7H), 6.83 (t, 2H), 6.72 - 6.64 (m, 5H), 6.52 (d, 2H), 6.38 (s, 1 H), 6.30 (d, 2H).

### 2. Herstellung von organischen Elektrolumineszenzvorrichtungen, die erfindungsgemäße Mischungen enthalten

Die Herstellung von OLEDs erfolgte nach dem im folgenden skizzierten allgemeinen Verfahren. Dieses musste natürlich im Einzelfall auf die jeweiligen Gegebenheiten (z. B. Schichtdickenvariation, um optimale Effizienz bzw. Farbe zu erreichen) angepasst werden.

Erfindungsgemäße Elektrolumineszenzvorrichtungen können beispielsweise wie folgt dargestellt werden:
1. ITO beschichtetes Substrat: Als Substrat wird bevorzugt mit ITO beschichtetes Glas verwendet, das einen möglichst niedrigen Gehalt bzw. keine ionischen Verunreinigungen enthält, wie z. B. Flachglas von den Firmen Merck-Balzers oder Akaii. Es können aber auch andere mit ITO beschichtete transparente Substrate, wie z. B. flexible Kunststoffolien oder Laminate verwendet werden. Das ITO muss eine möglichst hohe Leitfähigkeit mit einer hoher Transparenz verbinden. ITO-Schichtdicken zwischen 50 und 200 nm haben sich als besonders geeignet herausgestellt. Die ITO Beschichtung muss möglichst flach, bevorzugt mit einer Rauhigkeit unter 2 nm, sein. Die Substrate werden zunächst mit einer 4%igen Dekonex-Lösung in entionisierten Wasser vorgereinigt. Danach wird das mit ITO beschichtete Substrat entweder mindestens 10 Minuten mit Ozon oder einige Minuten mit Sauerstoffplasma behandelt oder kurze Zeit mit einer Exzimer-Lampe bestrahlt.
2. Lochinjektionsschicht (Hole Injection Layer = HIL): Als HIL wird entweder ein Polymer oder eine niedermolekulare Substanz verwendet. Besonders geeignet sind die Polymere Polyanilin (PANI) oder Polythiophen (PEDOT) und deren Derivate. Es handelt sich meist um 1 bis 5%ige wäßrige Dispersionen, welche in dünnen Schichten zwischen 20 und 200 nm, bevorzugt zwischen 40 und 150 nm Schichtdicke auf das ITO-Substrat durch Spincoaten, Inkjet-Drucken oder andere Beschichtungsverfahren aufgebracht werden. Danach werden die mit PEDOT oder PANI beschichteten ITO-Substrate getrocknet. Für die Trocknung bieten sich mehrere Verfahren an. Herkömmlich werden die Filme im Trockenofen 1 bis 10 Minuten zwischen 110 und 200 °C, bevorzugt zwischen 150 und 180 °C, getrocknet. Aber auch neuere Trocknungsverfahren, wie z. B. Bestrahlung mit IR-(Infrarot)-Licht, führen zu sehr guten Resultaten, wobei die Bestrahlungsdauer im allgemeinen weniger als einige Sekunden dauert. Als niedermolekulares Material werden bevorzugt dünne Schichten, zwischen 5 und 30 nm, Kupferphthalocyanin (CuPc) verwendet. Herkömmlich wird CuPc in Vakuum-Sublimationsanlagen aufgedampft. Alle HIL müssen nicht nur sehr gut Löcher injizieren, sondern auch sehr gut auf ITO und Glas haften; dies ist sowohl für CuPc als auch für PEDOT und PANI der Fall. Eine besonders niedrige Absorption im sichtbaren Bereich und damit eine hohe Transparenz, welches eine weitere notwendige Eigenschaft für die HIL ist, zeigen PEDOT und PANI.
3. Eine oder mehrere Lochtransportschichten (Hole Transport Layer = HTL): Bei den meisten OLEDs sind eine oder mehrere HTLs Voraussetzung für eine gute Effizienz und hohe Stabilität. Dabei erreicht man mit einer Kombination von zwei Schichten beispielsweise bestehend aus Triarylaminen wie MTDATA (4,4',4"-Tris(N-3-methylphenyl-N-phenyl-amino)-triphenylamin) oder NaphDATA (4,4',4"-Tris(N-1-naphthyl-N-phenyl-amino)-triphenylamin) als erste HTL und NPB (N,N'-Di(naphth-1-yl)-N,N'-diphenyl-benzidin) oder Spiro-TAD (Tetrakis-2,2',7,7'-diphenylamino-spiro-9,9'-bifluoren) als zweite HTL sehr gute Ergebnisse. MTDATA oder NaphDATA bewirken eine Erhöhung der Effizienz in den meisten OLEDs um ca. 20 - 40 %; wegen der höheren Glastemperatur T₉ wird NaphData (T_{g} = 130 °C) gegenüber MTDATA (T_{g} = 100 °C) bevorzugt. Als zweite Schicht wird Spiro-TAD (Tg = 130 °C) wegen der höheren T_{g} gegenüber NPB (Tg = 95 °C) bevorzugt. MTDATA bzw. NaphDATA haben eine Schichtdicke zwischen 5 und 100 nm, bevorzugt zwischen 10 und 60 nm, besonders bevorzugt zwischen 15 und 40 nm. Für dickere Schichten benötigt man etwas höhere Spannungen, um die gleiche Helligkeit zu erreichen; gleichzeitig verringert sich aber auch die Anzahl der Defekte. Spiro-TAD bzw. NPB haben eine Schichtdicke zwischen 5 und 150 nm, bevorzugt zwischen 10 und 100 nm, besonders bevorzugt zwischen 20 und 60 nm. Mit zunehmender Schichtdicke von NPB und den meisten anderen Triarylaminen benötigt man höhere Spannungen für gleiche Helligkeiten. Die Schichtdicke von Spiro-TAD hat jedoch nur einen geringfügigen Einfluss auf die Strom-Spannungs-Elektrolumineszenz-Kennlinien, d. h. die benötigte Spannung, um ein bestimmte Helligkeit zu erreichen, hängt nur geringfügig von der Spiro-TAD-Schichtdicke ab. Anstelle von niedermolekularen Triarylaminen können auch hochmolekulare Triarylamine verwendet werden. Es handelt sich meist um 0.1 bis 30%ige Lösungen, welche in dünnen Schichten zwischen 20 und 500 nm, bevorzugt zwischen 40 und 150 nm Schichtdicke auf das ITO-Substrat oder die HIL (z. B. PEDOT- oder PANI-Schicht) durch Spincoaten, Inkjet-Drucken oder andere Beschichtungsverfahren aufgebracht werden.
4. Emissionsschicht (Emission Layer = EML): Diese Schicht kann teilweise mit den Schichten 3 und/oder 5 zusammenfallen. Sie besteht z. B. aus einem niedermolekularen Matrixmaterial und einem niedermolekularen Gastmaterial, dem phosphoreszierenden Dotanden, wie beispielsweise CBP oder eines der oben beschriebenen Matrixmaterialien A als Matrixmaterial und Ir(PPy)₃ als Dotand. Gute Resultate erreicht man bei einer Konzentration von 5 - 30 % Ir(PPy)₃ in CBP oder in einem der oben beschriebenen Matrixmaterialien A bei einer EML-Schichtdicke zwischen 10 und 100 nm, bevorzugt zwischen 10 und 50 nm. Anstelle von niedermolekularen lichtemittierenden Verbindungen können auch hochmolekulare lichtemittierende Verbindungen (Polymere) verwendet werden, wobei eine oder auch beide Komponenten des Wirts-Gast-Systems hochmolekular sein können.
5. Eine Elektronentransport- und Lochblockierschicht (Hole Blocking Layer = HBL): Als HBL-Material haben sich besonders BCP (2,9-Dimethyl-4,7-diphenyl-1,10-phenanthrolin = Bathocuproin) oder BAlq als wirkungsvoll gezeigt. Anstelle von niedermolekularen HBLs können auch hochmolekulare HBLs verwendet werden. Es hat sich jedoch gezeigt, dass OLEDs, die erfindungsgemäße Mischungen enthalten, auch ohne eine solche Lochblockierschicht weiterhin sehr gute Ergebnisse zeigen. Deshalb wurde nicht in allen im folgenden beschriebenen Beispielen eine Lochblockierschicht verwendet.
6. Elektronentransportschicht (Electron Transport Layer = ETL): Als ETL-Materialien sind Metall-hydroxychinolate gut geeignet; besonders Aluminium-tris-8-hydroxychinolat (Alq₃) hat sich als einer der stabilsten Elektronenleiter herausgestellt. Anstelle von niedermolekularen ETLs können auch hochmolekulare ETLs verwendet werden. Es hat sich jedoch gezeigt, dass OLEDs, die erfindungsgemäße Mischungen enthalten, auch ohne eine solche Elektronentransportschicht weiterhin sehr gute Ergebnisse, insbesondere sehr niedrige Spannungen und hohe Leistungseffizienzen, zeigen. Deshalb wurde nicht in allen im folgenden beschriebenen Beispielen eine Elektronentransportschicht verwendet.
7. Elektroneninjektionsschicht (Electron Injection Layer = EIL): Eine dünne Schicht mit einer Schichtdicke zwischen 0.2 und 8 nm, bevorzugt zwischen 0.5 und 5 nm, bestehend aus einem Material mit einer hohen Dielektrizitätskonstanten, insbesondere anorganische Fluoride und Oxide, wie z. B. LiF, Li₂O, BaF₂, MgO, NaF, und weitere Materialien, hat sich als EIL als besonders gut herausgestellt. Speziell in Kombination mit Al führt diese zusätzliche Schicht zu einer deutlichen Verbesserung der Elektroneninjektion und damit zu verbesserten Resultaten bezüglich Lebensdauer, Quanten- und Leistungseffizienz.
8. Kathode: Hier werden in der Regel Metalle, Metallkombinationen oder Metallegierungen mit niedriger Austrittsarbeit verwendet, so z. B. Ca, Ba, Cs, K, Na, Mg, Al, In, Mg/Ag.
9. a) Herstellung dünner Schichten (2.-8.) niedermolekularer Verbindungen: Alle niedermolekularen Materialien der HIL, HTL, EML, HBL, ETL, EIL und Kathode werden in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft. Die Aufdampfraten können zwischen 0.01 und 10 nm/s, bevorzugt 0.1 und 1 nm/s, betragen. Neuere Verfahren wie die OPVD (Organic Physical Vapour Deposition) oder LITI (Light Induced Thermal Imaging) sind für die Beschichtung niedermolekularer Materialien ebenso geeignet, ebenso weitere Drucktechniken. Für dotierte Schichten hat die OPVD ein großes Potential, weil das Einstellen beliebiger Mischungsverhältnisse besonders gut gelingt. Ebenfalls lassen sich die Konzentrationen der Dotanden kontinuierlich verändern. Somit sind bei der OPVD die Voraussetzung für die Verbesserung der Elektrolumineszenz-Vorrichtung optimal. Wie oben beschrieben, kann die Herstellung der erfindungsgemäßen Vorrichtungen auch durch spezielle Druckverfahren (wie das genannte LITI) durchgeführt werden. Dies hat sowohl Vorteile hinsichtlich der Skalierbarkeit der Fertigung, als auch bezüglich der Einstellung von Mischungsverhältnissen in verwendeten Blend-Schichten. Hierfür ist es aber in aller Regel nötig, entsprechende Schichten (für LITI: Transfer-Schichten) zu präparieren, welche dann erst auf das eigentliche Substrat übertragen werden.
   b) Herstellung dünner Schichten (2.-6.) hochmolekularer Verbindungen (Polymere): Es handelt sich meist um 0.1 bis 30%ige Lösungen oder Dispersionen, welche in dünnen Schichten zwischen 10 und 500 nm, bevorzugt zwischen 10 und 80 nm Schichtdicke auf das ITO-Substrat oder darunterliegende Schichten durch Spincoaten, Inkjet-Drucken, LITI oder andere Beschichtungsverfahren und Drucktechniken aufgebracht werden.
10. Verkapselung: Eine effektive Einkapselung der organischen Schichten inklusive der EIL und der Kathode ist für organische Elektrolumineszenzvorrichtungen unerläßlich. Wenn das organische Display auf einem Glassubstrat aufgebaut ist, gibt es mehrere Möglichkeiten. Eine Möglichkeit ist das Verkleben des gesamten Aufbaus mit einer zweiten Glas- oder Metallplatte. Dabei haben sich Zwei-Komponenten- oder UV-härtende-Epoxykleber als besonders geeignet erwiesen. Dabei kann die Elektrolumineszenzvorrichtung vollständig oder aber auch nur am Rand verklebt werden. Wird das organische Display nur am Rand verklebt, kann man die Haltbarkeit zusätzlich verbessern, indem man einen sogenannten Getter hinzufügt. Dieser Getter besteht aus einem sehr hygroskopischen Material, insbesondere Metalloxide, wie z. B. BaO, CaO, usw., welches eindringendes Wasser und Wasserdämpfe bindet. Eine zusätzliche Bindung von Sauerstoff erreicht man mit Gettermaterialien, wie z. B. Ca, Ba, usw.. Bei flexiblen Substraten ist besonders auf eine hohe Diffusionsbarriere gegenüber Wasser und Sauerstoff zu achten. Hier haben sich insbesondere Laminate aus alternierenden dünnen Kunststoff- und anorganischen Schichten (z. B. SiOₓ oder SiNₓ) bewährt.

### 3. Device-Beispiele

Hier werden die Ergebnisse verschiedener OLEDs gegenübergestellt. Der grundlegende Aufbau, wie die verwendeten Materialien, Dotierungsgrad und ihre Schichtdicken, war für die Beispielexperimente zur besseren Vergleichbarkeit identisch. Es wurde ausschließlich das Wirtsmaterial in der Emitterschicht getauscht, und die Beispiele wurden mit unterschiedlichen Triplett-Emittern durchgeführt.

Das erste Beispiel beschreibt einen Vergleichsstandard nach dem Stand der Technik, bei dem die Emitterschicht aus dem Wirtsmaterial CBP und dem Gastmaterial Ir(PPy)₃ (synthetisiert nach WO 02/060910) besteht.

Des Weiteren wird eine OLED mit einer Emitterschicht bestehend aus dem Wirtsmaterial Bis(9,9'-spiro-bifluoren-2-yl)keton und dem Gastmaterial Ir(PPy)₃ beschrieben. Das zweite Beispiel beschreibt einen weiteren Vergleich zwischen CBP und Bis(9,9'-spiro-bifluoren-2-yl)keton (s. Beispiel 1) mit dem roten Emitter Ir(BTP)₃ (synthetisiert nach WO 02/060910). Das dritte Beispiel beschreibt zwei OLEDs, das eine Mal ein tiefroter Emitter Ir(piq)₃ mit Bis(9,9'-spiro-bifluoren-2-yl)keton und das andere Mal ein roter Emitter Ir(FMepiq)₃ mit Bis(9,9'-spiro-bifluoren-2-yl)keton.

Analog dem o. g. allgemeinen Verfahren wurden grün und rot emittierende OLEDs mit folgendem Aufbau erzeugt:
- PEDOT: 60 nm (aus Wasser aufgeschleudert; PEDOT bezogen von H. C. Starck; Poly-[3,4-ethylendioxy-2,5-thiophen])
- NaphDATA: 20 nm (aufgedampft; NaphDATA bezogen von SynTec; 4,4',4"-Tris(N-1-naphthyl-N-phenyl-amino)-triphenylamin
- S-TAD: 20 nm (aufgedampft; S-TAD hergestellt nach WO99/12888; 2,2',7,7'-Tetrakis(diphenylamino)-spirobifluoren)
- Emitter-Schicht:: CBP 20 nm (aufgedampft; CBP bezogen von ALDRICH und weiter aufgereinigt, schließlich noch zweimal sublimiert; 4,4'-Bis-(N-carbazolyl)biphenyl) (Vergleichsstandard)
- ODER:: Bis(9,9'-spiro-bifluoren-2-yl)keton 20 nm (aufgedampft, synthetisiert und aufgereinigt nach Beispiel 1), jeweils dotiert mit 10 % Triplett-Emitter
- Ir(PPy)₃: (aufgedampft)
ODER:
- Ir(BTP)₃: (aufgedampft)
ODER:
- Ir(piq)₃: (aufgedampft)
ODER:
- Ir(FMepiq)₃: (aufgedampft)

- Bathocuproin (BCP): 10 nm (aufgedampft; BCP bezogen von ABCR, verwendet wie erhalten; 2,9-Dimethyl-4,7-diphenyl-1,10-phenanthrolin); nicht in allen Beispielen verwendet
- AIQ₃: 10 nm (aufgedampft; AIQ₃ bezogen von SynTec; Tris(chinolinolato)aluminium(III)), nicht in allen Beispielen verwendet
- Ba-Al: 3 nm Ba, darauf 150 nm Al als Kathode.

Diese noch nicht optimierten OLEDs wurden standardmäßig charakterisiert; hierfür wurden die Elektrolumineszenzspektren, die Effizienz (gemessen in Cd/A) in Abhängigkeit von der Helligkeit, berechnet aus Strom-Spannungs-Helligkeit-Kennlinien (IUL-Kennlinien), und die Lebensdauer bestimmt.

Zur Übersicht sind im folgenden die verwendeten Triplett-Emitter und die verwendeten Wirtsmaterialien abgebildet:

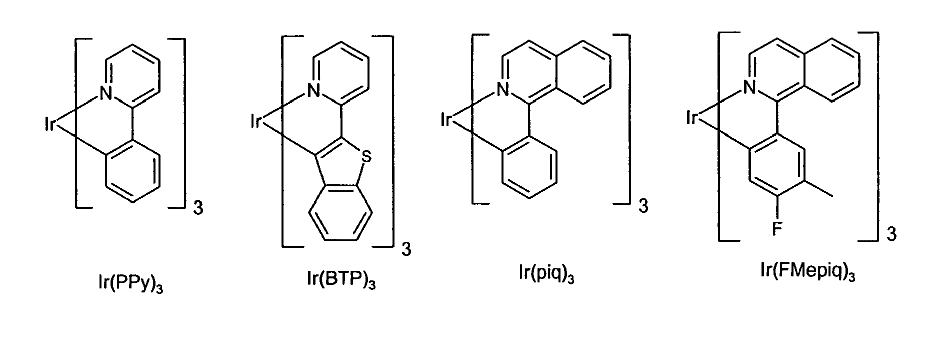

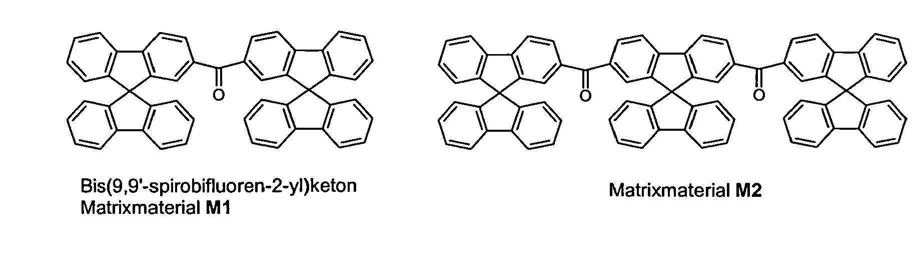

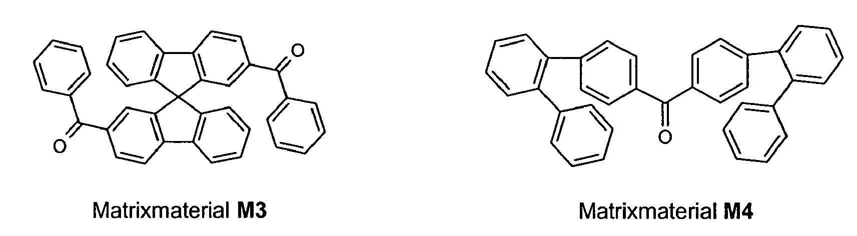

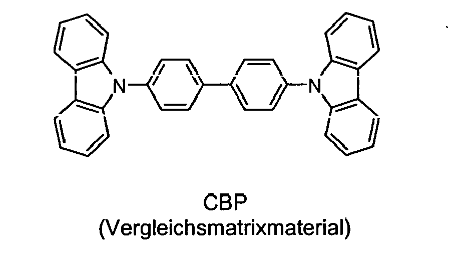

### Anwendungsbeispiel 1: Ir(PPy)₃

### Elektrolumineszenzspektren:

Die OLEDs, sowohl der Vergleichsstandard OLED mit CBP, als auch die OLED mit Bis(9,9'-spiro-bifluoren-2-yl)keton als Wirtsmaterial zeigen eine grüne Emission, resultierend aus dem Dotanden Ir(PPy)₃.

### Effizienz als Funktion der Helligkeit:

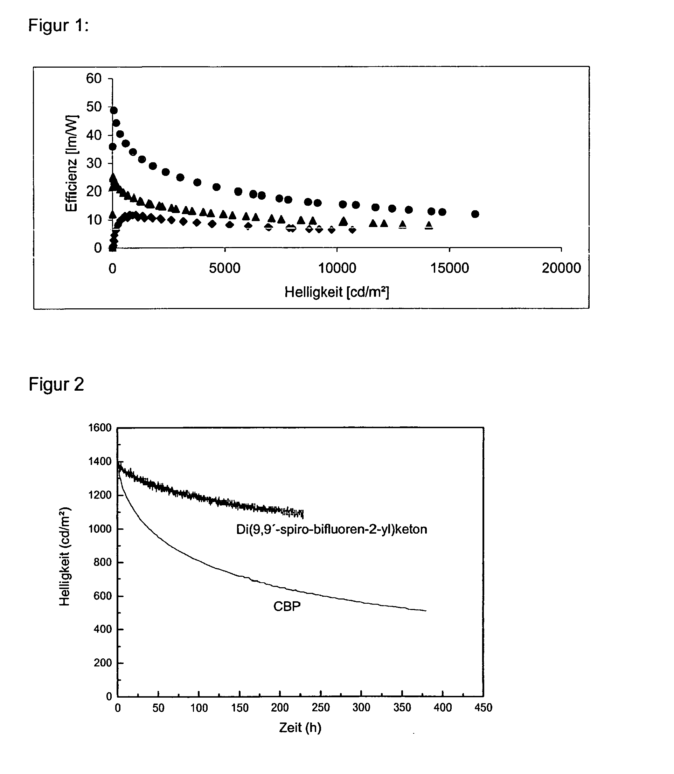

Für OLEDs hergestellt mit dem Wirtsmaterial CBP erhält man typischerweise eine maximale Effizienz von etwa 25 cd/A und für die Referenzleuchtdichte von 100 cd/m² werden 4.8 V benötigt. Im Gegensatz dazu zeigen OLEDs hergestellt mit dem Wirtsmaterial Bis(9,9'-spiro-bifluoren-2-yl)keton eine maximale Effizienz von über 30 cd/A, wobei die benötigte Spannung für die Referenzleuchtdichte von 100 cd/m² sogar auf 4.6 V sinkt. Ganz besonders hoch ist die Effizienz, wenn weder eine Lochblockierschicht (HBL) noch eine Elektronentransportschicht (ETL) verwendet wird und die dotierte Emissionsschicht (EML) bis an die Kathode reicht. Eine maximale Effizienz von über 35 cd/A wird erreicht, wobei die benötigte Spannung für die Referenzleuchtdichte von 100 cd/m² sogar unter 3 V sinkt. Besonders die Leistungseffizienz erhöht sich mit Verwendung von Bis(9,9'-spiro-bifluoren-2-yl)keton als Wirtsmaterial (▲) gegenüber CBP (◆) als Wirtsmaterial um 20% bis 100% (Fig. 1). Ganz besonders hohe Leistungseffizienzen bis 50 Im/W (●) erhält man, wenn weder eine Lochblockierschicht (HBL) noch eine Elektronentransportschicht (ETL) verwendet wird und die Dotierung der Emissionsschicht (EML) bis an die Kathode reicht.

### Lebensdauervergleich:

Die beiden Lebensdauerkurven (Fig. 2) mit CBP und mit Bis(9,9'-spirobifluoren-2-yl)keton als Wirtsmaterialien (beide hier verwendet mit Lochblockier- und Elektronentransportschicht) wurden zur besseren Vergleichbarkeit in derselben Figur dargestellt. Die Figur zeigt den Verlauf der Leuchtdichte, gemessen in cd/m², mit der Zeit. Als Lebensdauer bezeichnet man üblicherweise die Zeit, nach der nur noch 50 % der Anfangsleuchtdichte erreicht werden.

Man erhält mit CBP als Wirtsmaterial eine Lebensdauer von ca. 150 Stunden bei einer Anfangshelligkeit von 1400 cd/m², was einer beschleunigten Messung entspricht, da die Anfangshelligkeit deutlich über der Helligkeit liegt, die man für typische Aktiv-Matrix-angesteuerte Display-Anwendungen benötigt (250 cd/m²). Für Bis(9,9'-spiro-bifluoren-2-yl)keton erhält man bei derselben Anfangshelligkeit eine Lebensdauer von ca. 2000 Stunden, was einer Steigerung der Lebensdauer um etwa 1300 % entspricht; dies gilt auch, wenn weder eine Lochblockierschicht (HBL) noch eine Elektronentransportschicht (ETL) verwendet wird.

Aus diesen gemessenen Lebensdauern lassen sich nun Lebensdauern für eine Anfangshelligkeit von 250 cd/m² berechnen. Im Falle des Wirtsmaterial CBP erhält man lediglich eine Lebensdauer von 4700 Stunden, was deutlich unter den geforderten 10000 Stunden für eine Display-Anwendungen liegt. Im Gegensatz dazu erhält man mit Bis(9,9'-spirobifluoren-2-yl)keton eine Lebensdauer von über 60000 Stunden, was die Mindestanforderungen deutlich übertrifft.

### Anwendungsbeispiel 2 Ir(BTP)₃

Analoge Experimente wurden mit einem roten Triplettemitter Ir(BTP)₃ durchgeführt.

### Elektrolumineszenzspektren:

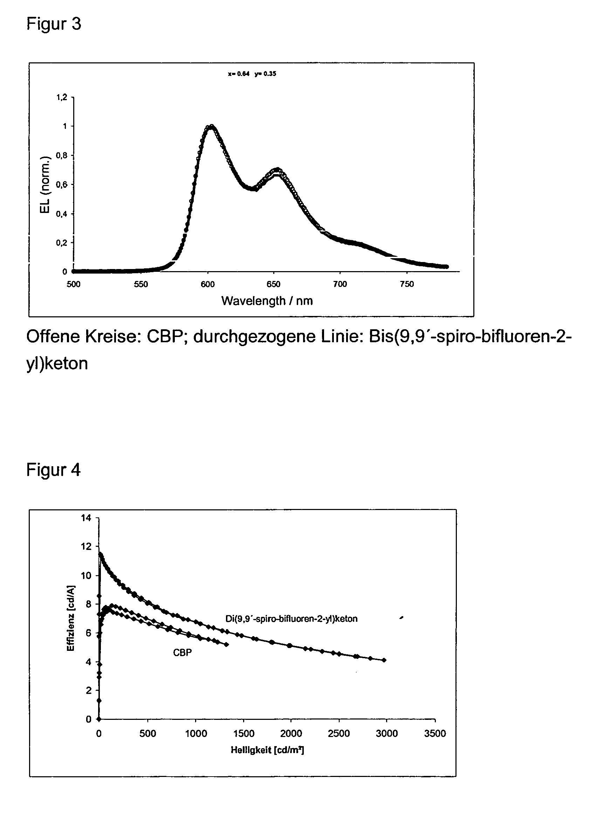

Die OLEDs, sowohl der Vergleichsstandard OLED mit CBP, als auch die OLED mit Bis(9,9'-spirobifluoren-2-yl)keton als Wirtsmaterial, zeigen eine rote Emission, resultierend aus dem Dotanden Ir(BTP)₃. Die beiden Spektren sind in Abb. 3 dargestellt.

### Effizienz als Funktion der Helligkeit:

Für OLEDs hergestellt mit dem Wirtsmaterial CBP erhält man typischerweise eine maximale Effizienz von etwa 8 cd/A und für die Referenzleuchtdichte von 100 cd/m² werden 6.2 V benötigt. Im Gegensatz dazu zeigen OLEDs hergestellt mit dem Wirtsmaterial Bis(9,9'-spirobifluoren-2-yl)keton eine maximale Effizienz von über 11 cd/A, wobei die benötigte Spannung für die Referenzleuchtdichte von 100 cd/m² sogar auf 5.2 V sinkt (Abb. 4).

### Lebensdauervergleich:

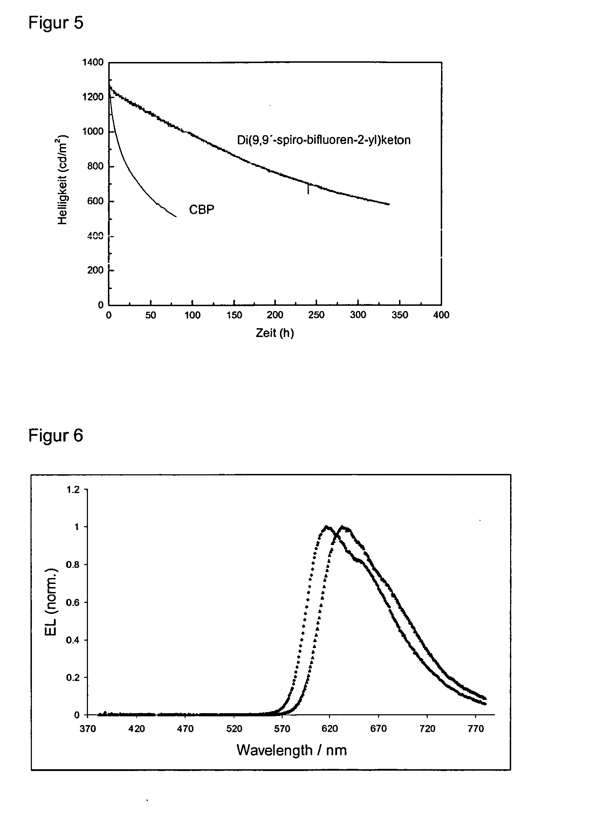

Die beiden Lebensdauerkurven (Abb. 5) wurden zur besseren Vergleichbarkeit in derselben Figur dargestellt. Die Figur zeigt den Verlauf der Leuchtdichte, gemessen in cd/m², mit der Zeit.

Man erhält mit CBP als Wirtsmaterial eine Lebensdauer von ca. 53 Stunden bei einer Anfangshelligkeit von knapp 1300 cd/m², was auch in diesem Beispiel einer beschleunigten Messung entspricht. Mit Bis(9,9'-spirobifluoren-2-yl)keton erhält man bei derselben Anfangshelligkeit eine Lebensdauer von ca. 275 Stunden, was einer Steigerung der Lebensdauer um etwa 500 % entspricht.

Aus diesen gemessenen Lebensdauern lassen sich nun Lebensdauern für eine Anfangshelligkeit von 250 cd/m² berechnen. Im Falle des Wirtsmaterial CBP erhält man lediglich eine Lebensdauer von 1600 Stunden, was deutlich unter den geforderten 10000 Stunden für Display-Anwendungen liegt. Im Gegensatz dazu erhält man mit Bis(9,9'-spirobifluoren-2-yl)keton eine Lebensdauer von über 8200 Stunden, was nahe an die Mindestanforderung herankommt.

### Anwendungsbeispiel 3: Ir(piq)₃ und Ir(FMepiq)₃

Ebenfalls konnten Experimente mit einem tiefroten Triplettemitter Ir(piq)₃ mit Bis(9,9'-spiro-bifluoren-2-yl)keton und einem roten Triplettemitter Ir(FMepiq)₃ mit Bis(9,9'-spirobifluoren-2-yl)keton durchgeführt werden.

### Elektrolumineszenzspektren:

Die OLEDs zeigen eine tiefrote Emission und eine rote Emission, resultierend aus den Dotanden Ir(piq)₃ (A) und Ir(FMepiq)₃ (◆). Die beiden Spektren sind in Figur 6 dargestellt. Aus den Spektren ergeben sich für Ir(piq)₃ in Bis(9,9'-spirobifluoren-2-yl)keton (A) die CIE Farbkoordinaten x = 0.69; y = 0.31 und für Ir(FMepiq)₃ in Bis(9,9'-spirobifluoren-2-yl)keton (◆) x = 0.66; y = 0.34.

### Effizienz als Funktion der Helligkeit:

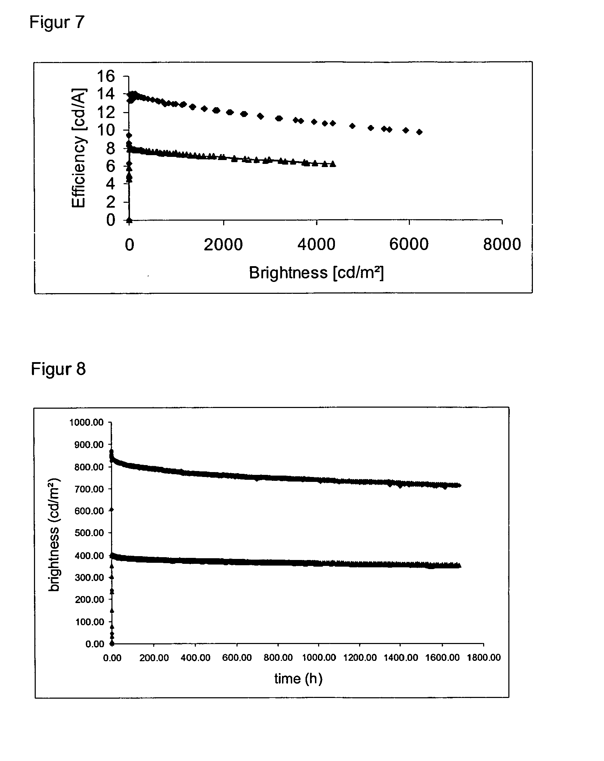

Sowohl Ir(piq)₃ (A) in Bis(9,9'-spirobifluoren-2-yl)keton als auch Ir(FMepiq)₃ (◆) in Bis(9,9'-spirobifluoren-2-yl)keton zeigen eine sehr hohe Effizienz von max. 8 cd/A (für Ir(piq)₃ (A) bei CIE Farbkoordinaten x = 0.69, y = 0.31) und 14 cd/A (für Ir(FMepiq)₃ (◆) bei CIE Farbkoordinaten x = 0.66, y = 0.34) (Abb. 7). Die benötigte Spannung für 100 cd/m² sank in beiden Fällen unter 6 V.

### Lebensdauer:

In Figur 8 ist die Lebensdauer von Ir(piq)₃ mit Bis(9,9'-spirobifluoren-2-yl)keton bei konstantem Strom von 10 mA/cm² bei einer Anfanshelligkeit von ca. 800 cd/m² und 5 mA/cm² bei einer Anfangshelligkeit von ca. 400 cd/m² dargestellt. Dabei erhält man einen Abfall der Helligkeit nach 1680 h bei 800 cd/m² Anfangshelligkeit von ca. 10% und nach 1680 h bei 400 cd/m² Anfangshelligkeit von ca. 5%. Eine Extrapolation ergibt eine Lebensdauer von ca. 5000 h bei 800 cd/m² Anfangshelligkeit und 20000 h bei 400 cd/m² Anfangshelligkeit. Für eine Anfangshelligkeit 200 cd/m² errechnet sich eine Lebensdauer von 80000 h. Ir(FMepiq)₃ in Bis(9,9'-spirobifluoren-2-yl)keton zeigt eine vergleichbare Lebensdauer.

Weitere Device-Beispiele sind in der folgenden Tabelle 2 zusammengefasst, wobei die Emission jeweils aus dem entsprechenden Emitter stammt.

**Tabelle 2:**

| **Experiment** | **EML** | **HBL** | **ETL** | **Max. Effizienz (cd/A)** | **Max. Leistungseffizienz (Im/W)** | **Spannung Spannung (V) bei 100cd/m**^{**2**} | **Lebensdauer (h) bei 10 mA/cm**^{**2**} |
|---|---|---|---|---|---|---|---|
| Vergleichsbeispiel zu 1) | CBP: 20% Ir(ppy)₃ (20 nm) | BCP (10 nm) | AIQ₃ (10 nm) | 25 | 12 | 4.8 | 150 |
| Beispiel 1a) | M1: 20% Ir(ppy)₃ (20 nm) | BCP (10 nm) | AIQ₃(10 nm) | 30 | 25 | 4.6 | 400 |
| Beispiel 1b) | M1: 20% Ir(ppy)₃ (60 nm) | - | - | 35 | 50 | 3.0 | 370 |
| Beispiel 1c) | M3: 20% Ir(ppy)₃ (20 nm) | BCP (10 nm) | AlQ₃ (10 nm) | 28 | 20 | 4.5 | 250 |
| Beispiel 1d) | M4: 20% Ir(ppy)₃ (20 nm) | BCP (10 nm) | AIQ₃ (10 nm) | 39 | 22 | 4.9 | 220 |
| Beispiel 1e) | M4: 20% Ir(ppy)₃ (20 nm) | - | - | 42 | 32 | 4.5 | 200 |
| | | | | | | | |
| Vergleichsbeispiel zu 2) | CBP:20% Ir(BTP)₃ (20 nm) | BCP (10 nm) | AlQ₃ (10 nm) | 8 | 5 | 6.2 | 53 |
| Beispiel 2a) | M1:20% Ir(BTP)₃ (20 nm) | BCP (10 nm) | AIQ₃ (10 nm) | 11 | 8 | 5.2 | 275 |
| Beispiel 2b) | M1:20% Ir(BTP)₃ (20 nm) | - | - | 11 | 10 | 4.1 | 250 |
| | | | | | | | |
| Vergleichsbeispiel zu 3) | CBP:20% Ir(piq)₃ (30 nm) | BCP (10 nm) | AIQ₃ (10 nm) | 7 | 4 | 6.4 | 5000 (extrapoliert) |
| Beispiel 3a) | M1:20% Ir(piq)₃ (30 nm) | BCP (10 nm) | AlQ₃ (10 nm) | 8 | 6 | 5.8 | 20000 (extrapoliert) |
| Beispiel 3b) | M1:20% Ir(piq)₃ (30 nm) | - | - | 7 | 8 | 3.2 | 15000 (extrapoliert) |
| Beispiel 3c) | M2:20% Ir(piq)₃ (30 nm) | BCP (10 nm) | AlQ₃ (10 nm) | 7 | 5 | 6.1 | 20000 (extrapoliert) |
| | | | | | | | |
| Beispiel 4a) | M1: 20% Ir(FMepiq)₃ 30 nm) | BCP (10 nm) | AIQₛ(10 nm) | 14 | 9 | 5.9 | 80000 |
| Beispiel 4b) | M1: 20% Ir(FMepiq)₃ (30 nm) | - | - | 15 | 12 | 4 | 80000 |
| Beispiel 4c) Vergleich | CBP: 20% Ir(FMepiq)₃ (30 nm) | BCP (10 nm) | AIQ₃ (10 nm) | 10 | 6 | 7 | 10000 |

## Patentansprüche

1. Mischungen enthaltend
- mindestens ein Matrixmaterial A der Formel (1), 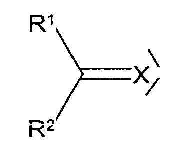 Formel (1)
wobei die Symbole und Indizes folgende Bedeutung haben:
X ist bei jedem Auftreten gleich oder verschieden O, S, Se oder NR³;
R¹, R², R³ ist gleich oder verschieden bei jedem Auftreten H, CN, eine geradkettige, verzweigte oder cyclische Alkyl-, Alkoxy- oder Alkylaminogruppe mit 1 bis 40 C-Atomen, wobei ein oder mehrere nicht benachbarte CH₂-Gruppen durch -C≡C-, C=O, C=S, C=Se, C=NR⁴, -O-, -S-, -NR⁵⁻oder -CONR⁶- ersetzt sein können und wobei ein oder mehrere H-Atome durch F, Cl, Br, I ersetzt sein können, oder ein aromatisches oder heteroaromatisches System mit 1 bis 40 C-Atomen, wobei ein oder mehrere H-Atome durch F, Cl, Br, I ersetzt sein können und das durch einen oder mehrere, nicht aromatische Reste R¹ substituiert sein kann; mit der Maßgabe, dass R¹=R²=R³ ungleich Wasserstoff ist;
R⁴, R⁹, R⁶ sind gleich oder verschieden bei jedem Auftreten H oder ein aliphatischer oder aromatischer Kohlenwasserstoffrest mit 1 bis 20 C-Atomen;
und
- mindestens ein zur Emission befähigtes Emissionsmaterial B, welches eine Verbindung ist, die bei geeigneter Anregung Licht emittiert und mindestens ein Element der Ordungszahl größer 20 enthält.

2. Mischung nach Anspruch 1, **dadurch gekennzeichnet, dass** für die Verbindung gemäß Formel (1) gilt:
X ist gleich oder verschieden bei jedem Auftreten O, S oder NR³,
R¹, R², R³ ist gleich oder verschieden bei jedem Auftreten H, eine geradkettige, verzweigte oder cyclische Alkylgruppe mit 1 bis 40 C-Atomen ohne H-Atome in α-Position zur Keto- bzw. Iminfunktion, wobei ein oder mehrere nicht benachbarte CH₂-Gruppen durch -C≡C-, C=O, C=NR⁴, -O-, -S-, -NR⁵- oder -CONR⁶- ersetzt sein können und wobei ein oder mehrere H-Atome durch F, Cl, Br, I ersetzt sein können, oder ein ein aromatisches oder heteroaromatisches System mit 1 bis 40 C-Atomen, wobei ein oder mehrere H-Atome durch F, Cl, Br, I ersetzt sein können und das durch einen oder mehrere, nicht aromatische Reste R¹ substituiert sein kann,
R⁴, R⁵, R⁶ sind wie unter Anspruch 1 beschrieben.

3. Mischungen enthaltend
- mindestens ein Matrixmaterial A der Formel (10) bis (15), 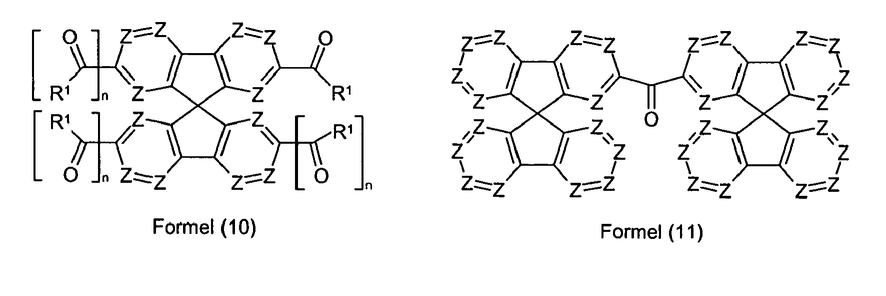 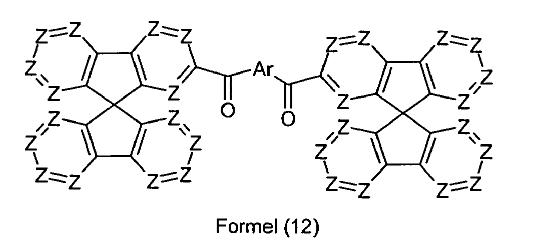 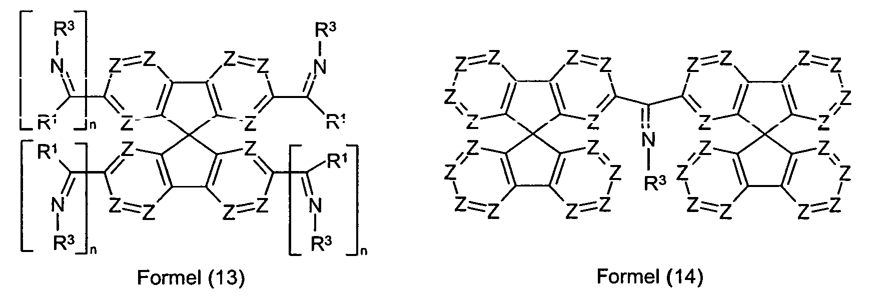 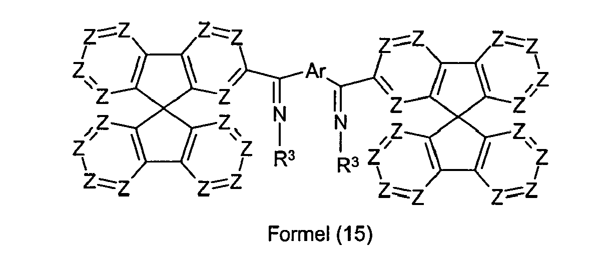 wobei R⁴, R⁵ und R⁶ dieselbe Bedeutung haben, wie unter Anspruch 1 beschrieben, und für die weiteren Symbole und Indizes gilt:
Ar ist bei jedem Auftreten gleich oder verschieden ein aromatisches oder heteroaromatisches System mit 2 bis 40 C-Atomen, wobei ein oder mehrere H-Atome durch F, Cl, Br, I ersetzt sein können und die durch einen oder mehrere, nicht aromatische Reste R¹ substituiert sein kann, wobei mehrere Substituenten R¹, sowohl am selben Ring als auch an unterschiedlichen Ringen zusammen wiederum ein weiteres mono- oder polycyclisches, aliphatisches oder aromatisches Ringsystem aufspannen können;
R¹, R², R³ ist gleich oder verschieden bei jedem Auftreten H, CN, eine geradkettige, verzweigte oder cyclische Alkyl-, Alkoxy- oder Alkylaminogruppe mit 1 bis 40 C-Atomen, wobei ein oder mehrere nicht benachbarte CH₂-Gruppen durch -R⁴C=CR⁴-, -C≡C-, C=O, C=S, C=Se, C=NR⁴, -O-, -S-, -NR⁵- oder -CONR⁶- ersetzt sein können und wobei ein oder mehrere H-Atome durch F, Cl, Br, I ersetzt sein können, oder ein aromatisches oder heteroaromatisches System mit 1 bis 40 C-Atomen, wobei ein oder mehrere H-Atome durch F, Cl, Br, I ersetzt sein können und das durch einen oder mehrere, nicht aromatische Reste R¹ substituiert sein kann, wobei mehrere Substituenten R¹ und/oder R¹, R² sowohl am selben Ring als auch an den beiden unterschiedlichen Ringen zusammen wiederum ein weiteres mono- oder polycyclisches, aliphatisches oder aromatisches Ringsystem aufspannen können; mit der Maßgabe, dass R¹=R²=R³ ungleich Wasserstoff ist;
Z ist gleich oder verschieden bei jedem Auftreten CR¹ oder N, bevorzugt CR¹;
n ist bei jedem Auftreten gleich oder verschieden 0 oder 1;
und
- mindestens ein zur Emission befähigtes Emissionsmaterial B, welches eine Verbindung ist, die bei geeigneter Anregung Licht emittiert und mindestens ein Element der Ordungszahl größer 20 enthält.

4. Mischung nach einem oder mehreren der Ansprüche 1 bis 3, **dadurch gekennzeichnet, dass** als Emitter B mindestens eine Verbindung eingesetzt wird, die bei geeigneter Anregung Licht emittiert und mindestens ein Atom der Ordungszahl größer 38 und kleiner 84 enthält.

5. Mischung nach einem oder mehreren der Ansprüche 1 bis 4, **dadurch gekennzeichnet, dass** als Emitter B mindestens eine Verbindung der Formel (16) bis (19) eingesetzt wird, 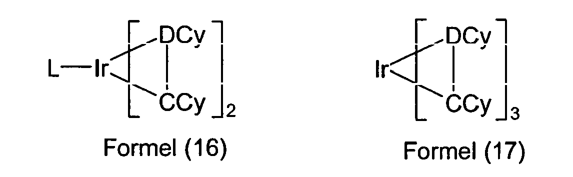 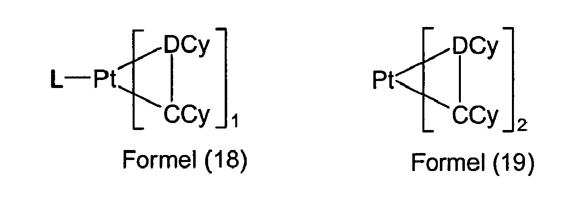 worin die Symbole R⁴ bis R⁶ dieselbe Bedeutung haben, wie in Anspruch 1 beschrieben, und für die verwendeten Symbole gilt:
DCy ist bei jedem Auftreten gleich oder verschieden eine cyclische Gruppe, die mindestens ein Donoratom enthält, über welches die cyclische Gruppe an das Metall gebunden ist, und die wiederum ein oder mehrere Substituenten R⁹ tragen kann; die Gruppen DCy und CCy sind über eine kovalente Bindung miteinander verbunden;
CCy ist bei jedem Auftreten gleich oder verschieden eine cyclische Gruppe, die ein Kohlenstoffatom enthält, über welches die cyclische Gruppe an das Metall gebunden ist und die wiederum ein oder mehrere Substituenten R⁹ tragen kann;
R⁹ ist gleich oder verschieden bei jedem Auftreten H, F, Cl, Br, I, NO₂, CN, eine geradkettige, verzweigte oder cyclische Alkyl- oder Alkoxygruppe mit 1 bis 40 C-Atomen, wobei ein oder mehrere nicht benachbarte CH₂-Gruppen durch -CR⁴=CR⁴-, -C≡C-, C=O, C=S, C=Se, C=NR⁴, -O-, -S-, -NR⁵- oder -CONR⁶- ersetzt sein können und wobei ein oder mehrere H-Atome durch F ersetzt sein können, oder ein aromatisches oder heteroaromatisches System mit 4 bis 40 C-Atomen, das durch einen oder mehrere, nicht aromatische Reste R⁹ substituiert sein kann; wobei mehrere Substituenten R⁹, sowohl am selben Ring als auch an den beiden unterschiedlichen Ringen zusammen wiederum ein weiteres mono- oder polycyclisches, aliphatisches oder aromatisches Ringsystem aufspannen können;
L ist bei jedem Auftreten gleich oder verschieden ein zweizähnig, chelatisierender Ligand.

6. Mischung nach einem oder mehreren der Ansprüche 1 bis 5, **dadurch gekennzeichnet, dass** das Matrixmaterial ein oder mehrere Polymere oder Dendrimere enthält.

7. Verbindungen gemäß Formel (10a) bis (15), 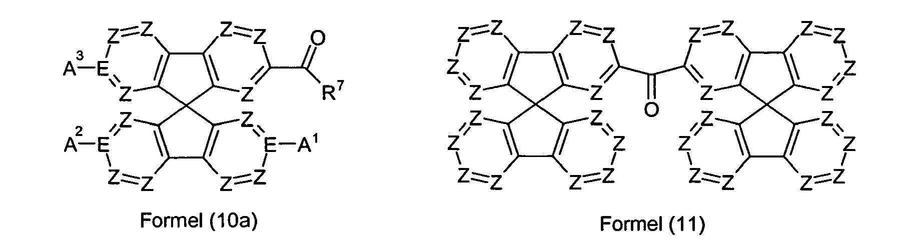 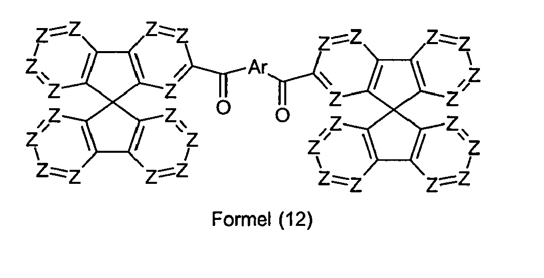 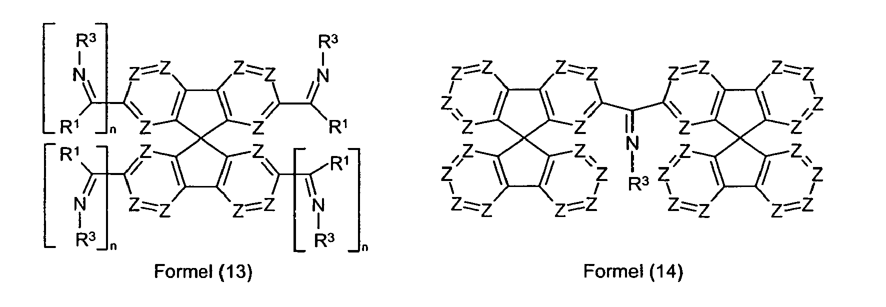 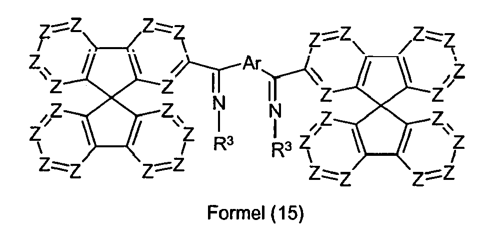 wobei die Symbole R⁴ bis R⁶ dieselbe Bedeutung haben, wie in Anspruch 1 beschrieben, und die Symbole Z, Ar und R¹ bis R³ dieselbe Bedeutung haben, wie unter Anspruch 3 beschrieben, und für die weiteren verwendeten Symbole gilt:
E ist bei jedem Auftreten gleich oder verschieden C oder N;
A¹ ist bei jedem Auftreten R⁸ oder CO-R⁷, wenn X = C ist, oder ein freies Elektronenpaar, wenn X = N ist;
A² ist bei jedem Auftreten R⁸ oder CO-R⁷, wenn X = C ist, oder ein freies Elektronenpaar, wenn X = N ist;
A³ ist bei jedem Auftreten R⁸ oder CO-R⁷, wenn X = C ist, oder ein freies Elektronenpaar, wenn X = N ist;
R⁷ ist bei jedem Auftreten gleich oder verschieden eine Alkyl-, Alkoxy- oder Alkylaminogruppe mit 1 bis 40 C-Atomen, in der ein oder mehrere CH₂-Gruppen durch -R⁴C=CR⁴-, -C≡C-, C=O, C=S, C=Se, C=NR⁴, -O-, -S-, -NR⁴- oder -CONR⁴- ersetzt sein können und in der auch ein oder mehrere H-Atome durch F, Cl, Br oder I ersetzt sein können, mit der Maßgabe, dass in α-Position zur Carbonylgruppe keine H-Atome gebunden sind, oder eine aromatische Gruppe, die gegebenenfalls mit Halogen, Alkyl, Trifluormethyl, Hydroxy, -SH, -S-Alkyl, Alkoxy, Nitro, Cyano, -COOH, -COOAlkyl, -NH₂, -NAlkyl, Benzyl oder Benzoyl substituiert sein kann, oder ein größeres aromatisches System mit 2 bis 40 C-Atomen, wobei ein oder mehrere H-Atome durch F, Cl, Br oder I ersetzt sein können und die durch ein oder mehrere nicht-aromatische Reste R¹ substituiert sein kann,
wobei mehrere Substituenten R¹ wiederum ein weiteres mono-oder polycyclisches, aliphatisches oder aromatisches Ring-system aufspannen können;
R⁸ ist gleich oder verschieden bei jedem Auftreten H, F, Cl, Br, I, CN, NO₂, eine geradkettige, verzweigte oder cyclische Alkylgruppe mit 1 bis 40 C-Atomen, wobei ein oder mehrere nicht benachbarte CH₂-Gruppen durch -R⁴C=CR⁴-, -C≡C-, C=S, C=Se, C=NR⁴, -O-, -S-, -NR⁴- oder -CONR⁴- ersetzt sein können und wobei ein oder mehrere H-Atome durch F, Cl, Br oder I ersetzt sein können, oder ein aromatisches oder hetero-aromatisches System mit 1 bis 40 C-Atomen, wobei ein oder mehrere H-Atome durch F, Cl, Br oder I ersetzt sein können und die durch einen oder mehrere, nicht-aromatische Reste R¹ substituiert sein kann, wobei mehrere Substituenten R¹ und/oder R¹/R⁴, sowohl am selben Ring als auch an den unterschiedlichen Ringen zusammen wiederum ein weiteres mono-oder polycyclisches, aliphatisches oder aromatisches Ringsystem aufspannen können;
mit der Maßgabe, dass für die Verbindung gemäß Formel (10a) für die beschriebenen Symbole nur folgende Kombinationen zugelassen sind, wobei R⁸ und R⁴ beliebig gemäß der Definition zu wählen sind:
• wenn R⁷ eine Alkylgruppe ohne α-H-Atome ist, sind die Symbole Z, E, A¹, A² und A³ beliebig gemäß der Definition zu wählen;
• wenn R⁷ eine aromatische Gruppe ist und mindestens ein Z für N steht, sind die Symbole E, A¹, A² und A³ beliebig gemäß der Definition zu wählen;
• wenn R⁷ eine aromatische Gruppe ist und mindestens ein Z für eine Gruppe CR¹ mit R¹ ungleich H steht, sind die Symbole E, A¹, A² und A³ beliebig gemäß der Definition zu wählen;
• wenn R⁷ eine aromatische Gruppe ist und alle Z für CH stehen und mindestens ein Symbol E für N steht, sind die Symbole A¹, A² und A³ beliebig gemäß der Definition zu wählen;
• wenn R⁷ eine aromatische Gruppe ist, alle Z für CH stehen und alle E für C stehen, muss mindestens eines der Symbole A¹, A² und/oder A³ für eine Gruppe R⁸ ungleich Alkyl stehen, während die beiden anderen Gruppen beliebig gemäß der Definition gewählt werden können;
• wenn R⁷ eine aromatische Gruppe ist, alle Z für CH stehen, alle E für C stehen und die beiden Symbole A¹ und A² beliebig gemäß der Definition gewählt sind, wobei mindestens eines der beiden Symbole für eine Gruppe ungleich H steht, dann steht das Symbol A³ für eine Gruppe CO-R⁷, wobei R⁷ hier beliebig gemäß der Definition zu wählen ist;
• wenn R⁷ ein größeres aromatisches System, wie beispielsweise Fluoren, Spirobifluoren, Triarylamin, etc., ist, dann sind die Symbole Z, E, A¹, A² und A³ beliebig gemäß der Definition zu wählen.

8. Elektronisches Bauteil, enthaltend mindestens eine Mischung nach einem oder mehreren der Ansprüche 1 bis 6 und/oder mindestens eine Verbindung gemäß Anspruch 7.

9. Elektronisches Bauteil nach Anspruch 8, **dadurch gekennzeichnet, dass** es sich um eine organische Leuchtdiode (OLED), eine organische integrierte Schaltung (O-IC), einen organischen Feld-Effekt-Transistor (OFET), einen organischen Dünnfilmtransistor (OTFT), eine organische Solarzelle (O-SC) oder eine organische Laserdiode (O-Laser) handelt.

10. Elektronisches Bauteil nach Anspruch 8 oder 9, **dadurch gekennzeichnet, dass** das elektronische Bauteil eine organische Leuchtdiode (OLED) ist, die mindestens eine Lochinjektionsschicht und/oder mindestens eine Lochtransportschicht und/oder mindestens eine Lochblockierschicht und/oder eine mindestens Elektronentransportschicht und/oder mindestens eine Elektroneninjektionsschicht und/oder weitere Schichten enthält und die mindestens eine erfindungsgemäße Mischung nach einem oder mehreren der Ansprüche 1 bis 6 in der Emissionsschicht enthält.

11. Elektronisches Bauteil nach einem oder mehreren der Ansprüche 8 bis 10, **dadurch gekennzeichnet, dass** eine Mischung nach einem oder mehreren der Ansprüche 1 bis 6 ohne Verwendung einer separaten Lochblockierschicht direkt an eine Elektronentransportschicht grenzt oder eine Mischung nach einem oder mehreren der Ansprüche 1 bis 6 ohne Verwendung einer separaten Lochblockierschicht und einer separaten Elektronentransportschicht direkt an eine Elektroneninjektionsschicht oder an die Kathode grenzt.
